(19) Europäisches Patentamt
European Patent Office
Office européen des brevets

(11) Veröffentlichungsnummer: **0 230 274**
**A2**

# (12) EUROPÄISCHE PATENTANMELDUNG

(21) Anmeldenummer: **87100460.2**

(22) Anmeldetag: **15.01.87**

(51) Int. Cl.⁴: **C 07 D 487/08**, C 07 D 519/00, C 07 D 471/08, C 07 D 498/08, C 07 D 498/04, C 07 D 215/56, A 61 K 31/53 // (C07D487/08, 241:00, 209:00),(C07D519/00, 487:00, 471:00),(C07D519/00, 498:00, 487:00),(C07D487/08, 209:00, 209:00),(C07D498/08, 307:00, 265:00),(C07D471/08, 221:00, 205:00)

(30) Priorität: **21.01.86 DE 3601567**

(43) Veröffentlichungstag der Anmeldung: **29.07.87**
**Patentblatt 87/31**

(84) Benannte Vertragsstaaten: **AT BE CH DE ES FR GB GR IT LI NL SE**

(71) Anmelder: **BAYER AG, Konzernverwaltung RP Patentabteilung, D-5090 Leverkusen 1 Bayerwerk (DE)**

(72) Erfinder: **Petersen, Uwe, Dr., Auf dem Forst 4, D-5090 Leverkusen 1 (DE)**
Erfinder: **Grohe, Klaus, Dr., Am Wasserturm 10, D-5068 Odenthal (DE)**
Erfinder: **Schenke, Thomas, Dr., Morgengraben 5, D-5000 Köln 80 (DE)**
Erfinder: **Hagemann, Hermann, Dr., Kandisky-Strasse 52, D-5090 Leverkusen 1 (DE)**
Erfinder: **Zeiler, Hans-Joachim, Dr., Elsbeeker Strasse 46, D-5620 Velbert 15 (DE)**
Erfinder: **Metzger, Karl Georg, Dr., Pahlkestrasse 75, D-5600 Wuppertal 1 (DE)**

## (54) 7-(Azabicycloalkyl)-chinoloncarbonsäure- und -naphthyridoncarbonsäure-Derivate.

(57) Die Erfindung betrifft neue 7-(Azabicycloalkyl)-chinoloncarbonsäure- und -naphthyridoncarbonsäure-Derivate der allgemeinen Formel (I)

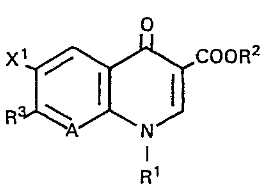

in der $R^1$, $R^2$, $R^3$, $X^1$ und A die in der Beschreibung angegebene Bedeutung haben, Verfahren zu ihrer Herstellung sowie diese enthaltende antibakterielle Mittel und Futterzusatzstoffe.

EP 0 230 274 A2

0230274

BAYER AKTIENGESELLSCHAFT 5090 Leverkusen, Bayerwerk
Konzernverwaltung RP
Patentabteilung Ad/AB

7-(Azabicycloalkyl)-chinoloncarbonsäure- und -naphthyridoncarbonsäure-Derivate

---

Die vorliegende Erfindung betrifft neue 7-(Azabicycloalkyl)-chinoloncarbonsäure- und -naphthyridoncarbonsäure-Derivate, Verfahren zu ihrer Herstellung sowie diese enthaltende antibakterielle Mittel und Futterzusatzstoffe.

Es ist bereits bekanntgeworden, daß 1-Ethyl-6-fluor-1,4-dihydro-4-oxo-7-(1-piperazinyl)-3-chinolincarbonsäuren (Deutsche Patentanmeldungen 2 084 097 und 2 939 786), 1-Cyclopropyl-6-fluor-1,4-dihydro-4-oxo-7-(1-piperazinyl)-3-chinolincarbonsäuren (Europäische Patentanmeldung 49 355, Deutsche Patentanmeldung 3 142 854), 1-Cyclopropyl-6-fluor-1,4-dihydro-4-oxo-7-(1-piperazinyl)-1,8-naphthyridin-3-carbonsäuren (Japanische Patentanmeldung 60 032 790), 9-Fluor-2,3-dihydro-3-methyl-7-oxo-10-(1-piperazinyl)-7H-pyrido[1,2,3-de][1,4]benzoxacin-6-carbonsäuren (Europäische Patentanmeldung 47 005), 1-Aryl-6-fluor-1,4-dihydro-4-oxo-7-(1-piperazinyl)-3-chinolincarbonsäuren (Europäische Patentanmeldung 131 839) und Azabicycloalkyl-chinoloncarbonsäuren (Europäische Patentanmeldung 159 174) antibakteriell wirksam sind.

Es wurde gefunden, daß die neuen 7-(Azabicycloalkyl)-chinoloncarbonsäure- beziehungsweise -naphthyridoncarbonsäure-Derivate der Formel (I)

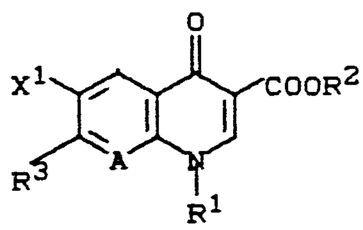

(I),

in welcher

$R^1$ für Methyl, Ethyl, Propyl, Isopropyl, Cyclopropyl, Vinyl, 2-Hydroxyethyl, 2-Fluorethyl, Methoxy, Amino, Methylamino, Dimethylamino, Phenyl, 4-Fluorphenyl, 2,4-Difluorphenyl,

$R^2$ für Wasserstoff, Alkyl mit 1 bis 4 Kohlenstoffatomen, (5-Methyl-2-oxo-1,3-dioxol-4-yl)-methyl,

$R^3$ für einen Rest der Formel

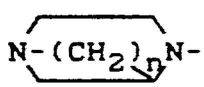 , 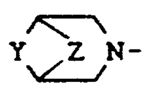 , 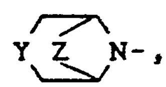 , 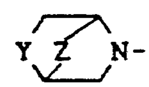 ,

in welcher

Y für $R^4$-N, O, S,

Z für $-(CH_2)_n-$, $-CH_2-O-CH_2$, $-CH_2-S-CH_2-$, $-CH_2-S-$,

$R^5$
|
$-CH_2-N-CH_2-$,

n für 1, 2 oder 3,

$R^4$ für Wasserstoff, gegebenenfalls durch Hydroxy substituiertes Alkyl, Alkenyl oder Alkinyl mit 1 bis 4 Kohlenstoffatomen, gegebenenfalls durch Nitro oder Amino substituiertes Benzyl, Oxoalkyl mit 2 bis 4 Kohlenstoffatomen oder (5-Methyl-2-oxo-1,3-dioxol-4-yl)-methyl,

$R^5$ für Wasserstoff oder Methyl steht,

$X^1$ für Fluor, Chlor oder Nitro und

A für N oder $C-R^6$ steht, worin
$R^6$ für Wasserstoff, Fluor, Chlor, Methyl oder Nitro steht oder auch gemeinsam mit $R^1$ eine Brücke der Struktur

$-O-CH_2-CH-CH_3$, $-S-CH_2-CH-CH_3$ oder $-CH_2-CH_2-CH-CH_3$

bilden kann,

mit der Maßgabe, daß für den Fall, daß $X^1$ für Fluor, $R^2$ für H und A für CF oder N stehen und $R^1$ für Ethyl, Cyclopropyl, Fluorethyl oder Vinyl steht oder $R^6$ mit $R^1$ eine Brücke der Struktur $-O-CH_2-CH-CH_3$ bildet, n ungleich 2 ist und $R^3$ nicht für den Rest

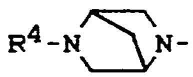 stehen kann, sowie ausgenommen die Verbindung 7-(2,5-Diazabicyclo[2,2,2]oct-2-yl)-1-ethyl-6-fluoro-1,4-dihydro-4-oxo-chinolincarbonsäure,

und deren pharmazeutisch verwendbaren Hydrate und Säureadditionssalze sowie die Alkali-, Erdalkali-, Silber- und Guanidiniumsalze der zugrundeliegenden Carbonsäuren eine hohe antibakterielle Wirkung insbesondere im grampositiven Bereich aufweisen.

Sie eignen sich daher als Wirkstoffe für die Human- und Veterinärmedizin, wobei zur Veterinärmedizin auch die Behandlung von Fischen zur Therapie oder Vorbeugung bakterieller Infektionen zu zählen ist.

Weiterer Gegenstand der Erfindung sind 7-(Azabicycloalkyl)-chinoloncarbonsäure- beziehungsweise -napthyridóncarbonsäure-Derivate der Formel (I)

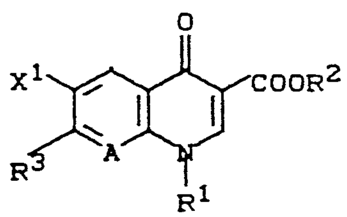

(I),

in welcher

$R^1$ für Methyl, Ethyl, Propyl, Isopropyl, Cyclopropyl, Vinyl, 2-Hydroxyethyl, 2-Fluorethyl, Methoxy, Amino, Methylamino, Dimethylamino, Phenyl, Fluorphenyl, 2,4-Difluorphenyl,

$R^2$ für Wasserstoff, Alkyl mit 1 bis 4 Kohlenstoffatomen, (5-Methyl-2-oxo-1,3-dioxol-4-yl)-methyl,

$R^3$ für einen im Ringsystem durch Hydroxy oder Methyl ein- oder mehrfach substituierten Rest der Formel

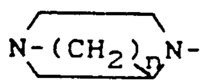 , 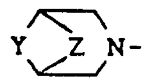 , 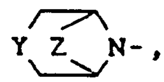 , 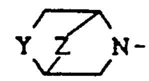 ,

in welcher

Y für $R^4$-N, O, S,

Z für $-(CH_2)_n-$, $-CH_2-O-CH_2-$, $-CH_2-S-CH_2-$, $-CH_2-S-$,

$$-CH_2-\underset{}{\overset{R^5}{\overset{|}{N}}}-CH_2-,$$

n für 1, 2 oder 3,

$R^4$ für Wasserstoff, gegebenenfalls durch Hydroxy substituiertes Alkyl, Alkenyl oder Alkinyl mit 1 bis 4 Kohlenstoffatomen, gegebenenfalls durch Nitro oder Amino substituiertes Benzyl, Oxoalkyl mit 2 bis 4 Kohlenstoffatomen oder (5-Methyl-2-oxo-1,3-dioxol-4-yl)-methyl und

$R^5$ für Wasserstoff oder Methyl steht,

$X^1$ für Fluor, Chlor oder Nitro und

A für N oder C-$R^6$ steht, worin
$R^6$ für Wasserstoff, Fluor, Chlor, Methyl oder Nitro steht oder auch gemeinsam mit $R^1$ eine Brücke der Struktur

$-O-CH_2-\underset{|}{CH}-CH_3$, $-S-CH_2-\underset{|}{CH}-CH_3$ **oder**

$-CH_2-CH_2-\underset{|}{CH}-CH_3$

**bilden kann**

und deren pharmazeutisch verwendbare Hydrate und Säureadditionssalze sowie die Alkali-, Erdalkali-, Silber- und Guanidiniumsalze der zugrundeliegenden Carbonsäuren.

**Bevorzugt sind diejenigen Verbindungen der Formel (I), in denen**

**$R^1$ für Methyl, Ethyl, Cyclopropyl, 2-Hydroxyethyl, Vinyl, 2-Fluorethyl, Methoxy, Methylamino, Phenyl, 4-Fluorphenyl, 2,4-Difluorphenyl,**

**$R^2$ für Wasserstoff, Methyl, Ethyl,**

$R^3$ für einen Rest der Formel

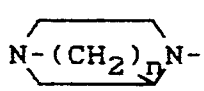 , 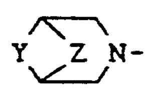 , 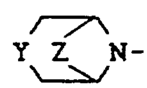 , 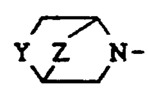 ,

in welcher

Y für $R^4$-N, O,

Z für $-(CH_2)_n-$, $-CH_2-O-CH_2-$, $-CH_2-N(R^5)-CH_2-$,

n für 1, 2 oder 3,

$R^4$ für Wasserstoff, gegebenenfalls durch Hydroxy substituiertes Alkyl, Alkenyl oder Alkinyl mit 1 bis 3 Kohlenstoffatomen, gegebenenfalls durch Nitro oder Amino substituiertes Benzyl oder Oxoalkyl mit 2 bis 4 Kohlenstoffatomen und

$R^5$ für Wasserstoff oder Methyl steht,

$X^1$ für Fluor, Chlor oder Nitro und

A für N oder C-$R^6$ steht, worin
$R^6$ für Wasserstoff, Fluor, Chlor, Methyl oder Nitro steht oder auch gemeinsam mit $R^1$ eine Brücke der Struktur

$-O-CH_2-\underset{|}{C}H-CH_3$ oder $-CH_2-CH_2-\underset{|}{C}H-CH_3$

bilden kann,

mit der Maßgabe, daß für den Fall, daß $X^1$ für Fluor, $R^2$ für H und A für CF oder N stehen und $R^1$ für Ethyl, Cyclopropyl, Fluorethyl oder Vinyl steht oder $R^6$ mit $R^1$ eine Brücke der Struktur $-O-CH_2-\underset{|}{C}H-CH_3$ bildet, n ungleich 2 ist und $R^3$ nicht für den Rest

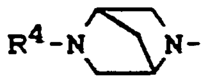 stehen kann, sowie ausgenommen die Verbindung 7-(2,5-Diazabicyclo[2.2.2]oct-2-yl)-1-ethyl-6-fluoro-1,4-dihydro-4-oxo-chinolincarbonsäure,

und deren pharmazeutisch verwendbaren Hydrate und Säureadditionssalze sowie die Alkali-, Erdalkali-, Silber- und Guanidiniumsalze der zugrundeliegenden Carbonsäuren.

Besonders bevorzugt sind diejenigen Verbindungen der Formel (I), in denen

$R^1$ für Ethyl, Cyclopropyl, 2-Fluorethyl, Vinyl, Methylamino, Phenyl, 4-Fluorphenyl, 2,4-Difluorphenyl,

$R^2$ für Wasserstoff, Methyl, Ethyl,

$R^3$ für einen Rest der Formel

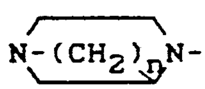 , 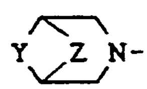 , 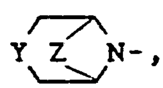 , 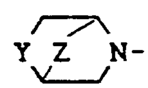 ,

in welcher

Y für $R^4$-N,

Z für $-(CH_2)_n-$,

n für 1 oder 2,

$R^4$ für Wasserstoff, gegebenenfalls durch Hydroxy substituiertes Alkyl, Alkenyl oder Alkinyl mit 1 bis 3 Kohlenstoffatomen, gegebenenfalls durch Nitro oder Amino substituiertes Benzyl oder Oxoalkyl mit 2 bis 4 Kohlenstoffatomen und

$R^5$ für Wasserstoff oder Methyl steht,

$X^1$ für Fluor, Chlor oder Nitro und

A für N oder C-$R^6$ steht, worin $R^6$ für Wasserstoff, Fluor, Chlor oder Nitro steht oder auch gemeinsam mit $R^1$ eine Brücke der Struktur

$-O-CH_2-\underset{|}{C}H-CH_3$ oder $-CH_2-CH_2-\underset{|}{C}H-CH_3$

bilden kann,

mit der Maßgabe, daß für den Fall, daß $X^1$ für Fluor, $R^2$ für H und A für CF oder N stehen und $R^1$ für Ethyl, Cyclopropyl, Fluorethyl oder Vinyl steht oder $R^6$ mit $R^1$ eine Brücke der Struktur $-O-CH_2-\underset{|}{C}H-CH_3$ bildet, n ungleich 2 ist und $R^3$ nicht für den Rest

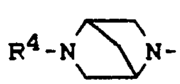 stehen kann, sowie ausgenommen die Verbindung 7-(2,5-Diazabicyclo[2.2.2]oct-2-yl)-1-ethyl-6-fluoro-1,4-dihydro-4-oxo-chinolincarbonsäure,

und deren pharmazeutisch verwendbaren Hydrate und Säureadditionssalze sowie die Alkali-, Erdalkali-, Silber- und Guanidiniumsalze der zugrundeliegenden Carbonsäuren.

Bevorzugt sind auch diejenigen Verbindungen der Formel (I), in denen

$R^1$ für Methyl, Ethyl, Cyclopropyl, 2-Hydroxyethyl, Vinyl, 2-Fluorethyl, Methoxy, Methylamino, Phenyl, 4-Fluorphenyl, 2,4-Difluorphenyl,

$R^2$ für Wasserstoff, Methyl, Ethyl,

$R^3$ für einen ein- oder mehrfach, bevorzugt 2-fach, durch Methyl substituierten Rest der Formel

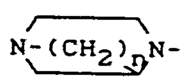 , 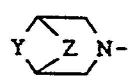 , 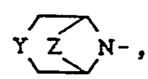 , 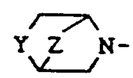 ,

in welcher

Y für $R^4$-N, O,

Z für $-(CH_2)_n-$, $-CH_2-O-CH_2-$, $-CH_2-N(R^5)-CH_2-$,

n für 1, 2 oder 3,

$R^4$ für Wasserstoff, gegebenenfalls durch Hydroxy substituiertes Alkyl, Alkenyl oder Alkinyl mit 1 bis 3 Kohlenstoffatomen, gegebenenfalls durch Nitro oder Amino substituiertes Benzyl oder Oxoalkyl mit 2 bis 4 Kohlenstoffatomen und

$R^5$ für Wasserstoff oder Methyl steht,

$X^1$ für Fluor, Chlor oder Nitro und

A für N oder C-$R^6$ steht, worin
$R^6$ für Wasserstoff, Fluor, Chlor, Methyl oder Nitro steht oder auch gemeinsam mit $R^1$ eine Brücke der Struktur

$-O-CH_2-\underset{|}{C}H-CH_3$, $-S-CH_2-\underset{|}{C}H-CH_3$ oder $-CH_2-CH_2-\underset{|}{C}H-CH_3$

bilden kann,

mit der Maßgabe, daß für den Fall, daß $X^1$ für Fluor, $R^2$ für H und A für CF oder N stehen und $R^1$ für Ethyl, Cyclopropyl, Fluorethyl oder Vinyl steht oder $R^6$ mit $R^1$ eine Brücke der Struktur $-O-CH_2-\underset{|}{C}H-CH_3$ bildet, n ungleich 2 ist und $R^3$ nicht für den Rest

und deren pharmazeutisch verwendbaren Hydrate und Säureadditionssalze sowie die Alkali-, Erdalkali-, Silber- und Guanidiniumsalze der zugrundeliegenden Carbonsäuren.

Besonders bevorzugt sind auch diejenigen Verbindungen der Formel (I), in denen

$R^1$ für Ethyl, Cyclopropyl, 2-Fluorethyl, Vinyl, Methylamino, Phenyl, 4-Fluorphenyl, 2,4-Difluorphenyl,

$R^2$ für Wasserstoff, Methyl, Ethyl,

$R^3$ für einen im Ringsystem ein- oder mehrfach, bevorzugt 2-fach, durch Methyl substituierten Rest der Formel

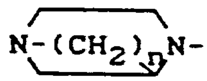 , 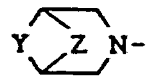 , 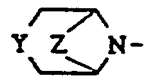 , 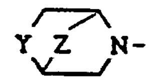 ,

in welcher

Y für $R^4$-N,

Z für $-(CH_2)_n-$,

n für 1 oder 2,

$R^4$ für Wasserstoff, gegebenenfalls durch Hydroxy substituiertes Alkyl, Alkenyl oder Alkinyl mit 1 bis 3 Kohlenstoffatomen, gegebenenfalls durch Nitro oder Amino substituiertes Benzyl oder Oxoalkyl mit 2 bis 4 Kohlenstoffatomen und

$R^5$ für Wasserstoff oder Methyl steht,

$X^1$ für Fluor, Chlor oder Nitro und

A für N oder C-$R^6$ steht, worin
$R^6$ für Wasserstoff, Fluor, Chlor oder Nitro steht oder auch gemeinsam mit $R^1$ eine Brücke der Struktur

$-O-CH_2-CH(-)-CH_3$ oder $-CH_2-CH_2-CH(-)-CH_3$

bilden kann,

und deren pharmazeutisch verwendbaren Hydrate und Säureadditionssalze sowie die Alkali-, Erdalkali-, Silber- und Guanidiniumsalze der zugrundeliegenden Carbonsäuren.

Weiterhin wurde gefunden, daß man die Verbindungen der Formel (I) erhält, wenn Verbindungen der Formel (II)

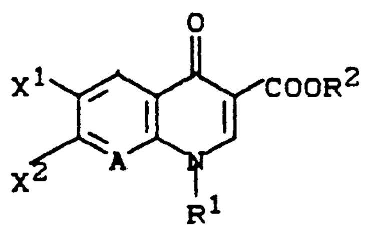

(II),

in welcher $R^1$, $R^2$, $X^1$ und A die oben angebenene Bedeutung haben und

$X^2$ für Halogen, insbesondere Fluor oder Chlor, steht,

mit Azabicycloalkanen der Formel (III)

$$R^3\text{-H} \quad \text{(III)},$$

in welcher $R^3$ die oben angegebene Bedeutung hat, gegebenenfalls in Gegenwart von Säurebindungsmitteln umsetzt (Methode A).

Erfindungsgemäße Verbindungen der Formel (I)

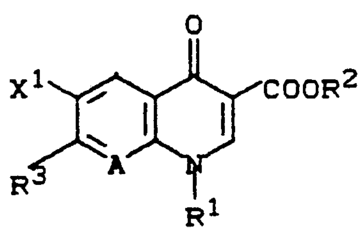

(I),

in welcher

$R^1$, $R^2$, A und $X^1$ die oben angegebene Bedeutung haben und

$R^3$ für einen im Ringsystem gegebenenfalls ein- oder mehrfach, bevorzugt 2-fach, durch Hydroxy oder Methyl substituierten Rest der Formel

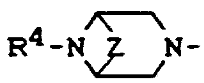 , 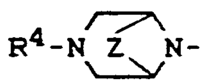 , 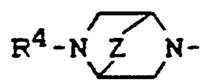 ,

in welcher

Z und $R^4$ die oben angegebene Bedeutung haben,

steht,

können auch erhalten werden, indem man eine Verbindung der Formel (IV)

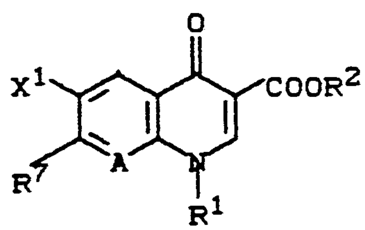 (IV),

in welcher

$R^1$, $R^2$, A und $X^1$ die oben angegebene Bedeutung haben und

$R^7$ für einen Rest der Formel

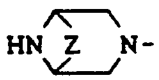 , 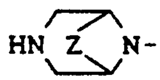 oder 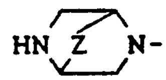 ,

in welcher

Z die oben angegebene Bedeutung hat, steht, mit Verbindungen der Formel (V)

$$R^4-X^3 \quad (V),$$

in welcher

$R^4$ die oben angegebene Bedeutung hat, aber nicht für Wasserstoff stehen kann, und

$X^3$ für Halogen, insbesondere Chlor, Brom oder Jod, steht, gegebenenfalls in Gegenwart von Säurebindungsmitteln umsetzt (Methode B).

Man erhält erfindungsgemäße Verbindungen der Formel (I), in der $R^4$ für $CH_3-CO-CH_2CH_2$ steht, auch durch Umsetzung einer Verbindung der Formel (IV) mit Methylvinylketon (Methode C).

Verwendet man bei der Umsetzung nach Methode A 1-Cyclopropyl-6,7-difluor-1,4-dihydro-4-oxo-3-chinolincarbonsäure und 1,4-Diazabicyclo[3.2.1]octan als Ausgangsstoffe, so kann der Reaktionsablauf durch das folgende Formelschema wiedergegeben werden:

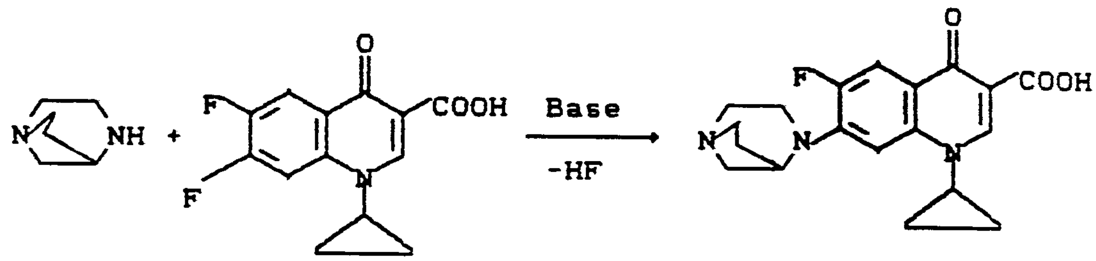


Verwendet man bei der Umsetzung nach Methode B 1-Cyclopropyl-7-(2,5-diazabicyclo[2.2.2]oct-2-yl)-6,8-difluor-1,4-dihydro-4-oxo-3-chinolincarbonsäure und Ethyliodid als Ausgangsstoffe, so kann der Reaktionsablauf durch das folgende Formelschema wiedergegeben werden:

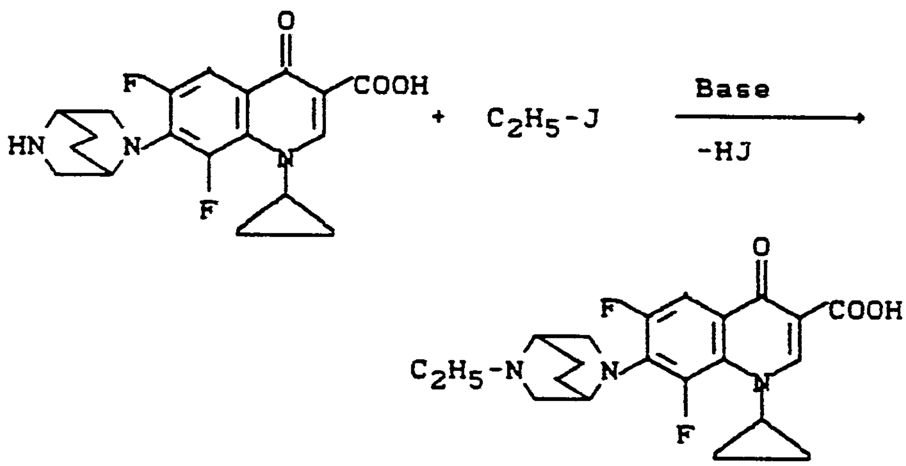


Verwendet man bei der Umsetzung nach Methode C 1-Cyclopropyl-7-(3,8-diazabicyclo[3.2.1]oct-3-yl)-6-fluor-1,4-dihydro-4-oxo-3-chinolincarbonsäure und Methylvinylketon als Ausgangsverbindungen, so kann der Reaktionsablauf durch das folgende Formelschema wiedergegeben werden:

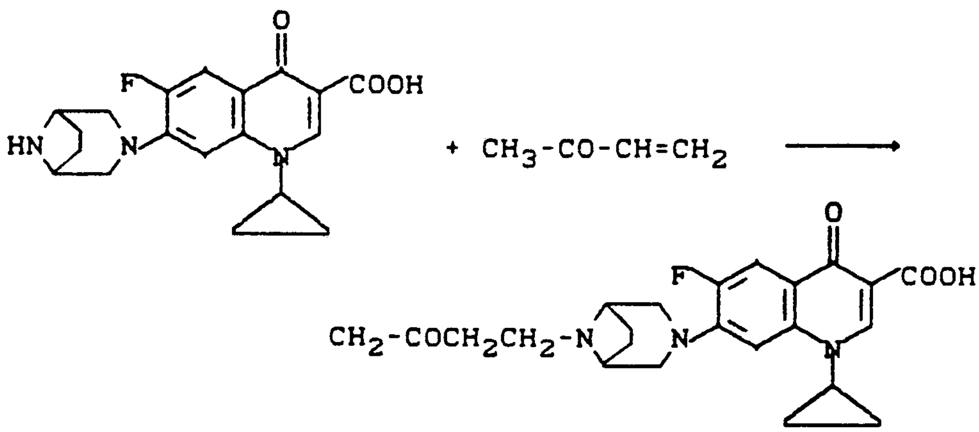

Die als Ausgangsstoffe verwendeten Verbindungen der Formel (II) sind bekannt oder können nach bekannten Methoden hergestellt werden. Als Beispiele seien genannt:

7-Chlor-1-cyclopropyl-6-fluor-1,4-dihydro-4-oxo-3-chinolincarbonsäure (Deutsche Patentanmeldung 3 142 854),

1-Cyclopropyl-6,7-difluor-1,4-dihydro-4-oxo-3-chinolincarbonsäure (Europäische Patentanmeldung 113 091),

6-Chlor-1-cyclopropyl-7,8-difluor-1,4-dihydro-4-oxo-3-chinolincarbonsäure (Deutsche Patentanmeldung 3 420 743),

8-Chlor-1-cyclopropyl-6,7-difluor-1,4-dihydro-4-oxo-3-chinolincarbonsäure (Deutsche Patentanmeldung 3 420 743),

1-Cyclopropyl-6,7,8-trifluor-1,4-dihydro-4-oxo-3-chinolincarbonsäure (Deutsche Patentanmeldung 3 318 145),

6,8-Dichlor-1-cyclopropyl-7-fluor-1,4-dihydro-4-oxo-3-chinolincarbonsäure (Deutsche Patentanmeldung 3 420 743),

7-Chlor-1-cyclopropyl-1,4-dihydro-6-nitro-4-oxo-3-chinolincarbonsäure (Europäische Patentanmeldung 113 091),

6,7-Dichlor-1-cyclopropyl-1,4-dihydro-4-oxo-3-chinolincarbonsäure (Europäische Patentanmeldung 113 091),

1-Cyclopropyl-6,7-difluor-1,4-dihydro-8-methyl-4-oxo-3-chinolincarbonsäure,

1-Cyclopropyl-7-chlor-6-fluor-1,4-dihydro-8-nitro-4-oxo-3-chinolincarbonsäure,

6,7-Difluor-1-ethyl-1,4-dihydro-4-oxo-3-chinolincarbonsäure,

7-Chlor-6-fluor-1-ethyl-1,4-dihydro-4-oxo-3-chinolincarbonsäure,

7-Chlor-6-fluor-1,4-dihydro-1-(2-hydroxyethyl)-4-oxo-3-chinolincarbonsäure,

6,7-Difluor-1-(2-fluorethyl)-1,4-dihydro-4-oxo-3-chinolincarbonsäure,

7-Chlor-6-fluor-1,4-dihydro-1-methoxy-4-oxo-3-chinolincarbonsäure,

7-Chlor-6-fluor-1,4-dihydro-1-methylamino-4-oxo-3-chinolincarbonsäure,

6,7-Difluor-1,4-dihydro-4-oxo-1-phenyl-3-chinolincarbonsäure,

7-Chlor-1-cyclopropyl-6-fluor-1,4-dihydro-4-oxo-1,8-naphthyridin-3-carbonsäure,

6,7-Dichlor-1-cyclopropyl-1,4-dihydro-4-oxo-1,8-naphthyridin-3-carbonsäure,

1-Cyclopropyl-6,7,8-trifluor-1,4-dihydro-4-oxo-3-chinolincarbonbonsäureethylester (Deutsche Patentanmeldung 3 318 145),

9,10-Difluor-2,3-dihydro-3-methyl-7-oxo-7H-pyrido-[1,2,3-de][1,4]benzoxacin-6-carbonsäure (Europäische Patentanmeldung 47 005),

8,9-Difluor-6,7-dihydro-5-methyl-1-oxo-1H,5H-benzo[i,j]-chinolicin-2-carbonsäure,

7-Chlor-6-fluor-1-phenyl-1,4-dihydro-4-oxo-1,8-naphthyridin-3-carbonsäure (Europäische Patentanmeldung 153 580),

7-Chlor-6-fluor-1-(4-fluorphenyl)-1,4-dihydro-4-oxo-1,8-naphthydrin-3-carbonsäure (Europäische Patentanmeldung 153 580),

6,7,8-Trifluor-1,4-dihydro-1-methylamino-4-oxo-3-chinolincarbonsäure (Deutsche Patentanmeldung 3 409 922),

1-Amino-6,7,8-trifluor-1,4-dihydro-4-oxo-3-chinolincarbonsäure (Deutsche Patentanmeldung 3 409 922),

6,7,8-Trifluor-1,4-dihydro-1-dimethylamino-4-oxo-3-chinolincarbonsäure (Deutsche Patentanmeldung 3 409 922),

7-Chlor-6-fluor-1,4-dihydro-8-nitro-4-oxo-1-phenyl-3-chinolincarbonsäure,

7-Chlor-6-fluor-1-(4-fluorphenyl)-1,4-dihydro-8-nitro-4-oxo-3-chinolincarbonsäure,

6,7-Difluor-1-(4-fluorphenyl)-1,4-dihydro-8-methyl-4-oxo-3-chinolincarbonsäure,

6-Chlor-7-fluor-1-(4-fluorphenyl)-1,4-dihydro-4-oxo-3-chinolincarbonsäure (Europäische Patentanmeldung 131 839),

6-Chlor-7-fluor-1-(2,4-difluorphenyl)-1,4-dihydro-4-oxo-3-chinolincarbonsäure (Europäische Patentanmeldung 131 839),

6,7,8-Trifluor-1-(4-fluorphenyl)-1,4-dihydro-4-oxo-3-chinolincarbonsäure (Europäische Patentanmeldung 154 780),

6,7,8-Trifluor-1-(2,4-difluorphenyl)-1,4-dihydro-4-oxo-3-chinolincarbonsäure (Europäische Patentanmeldung 154 780),

6,7,8-Trifluor-1,4-dihydro-4-oxo-1-phenyl-3-chinolincarbonsäure (Europäische Patentanmeldung 154 780),

7-Chlor-1-ethyl-6-fluor-1,4-dihydro-4-oxo-1,8-naphthyridin-3-carbonsäure,

6,7-Difluor-1,4-dihydro-4-oxo-1-vinyl-3-chinolincarbonsäure.

Die als Ausgangsverbindungen verwendeten Azabicycloalkane der Formel (III) sind zum Teil bekannt oder können nach bekannten Verfahren hergestellt werden. Die Verbindungen können als Racemate oder nach Auftrennung in die Enantiomeren als enantiomeren-reine Azabicycloalkane eingesetzt werden. Als Beispiele seien genannt:

1,4-Diazabicyclo[3.2.1]octan [US-Pat. 3 281 423, J. Med. Chem. 20, 1333 (1977)],

8-Methyl-3,8-diazabicyclo[3.2.1]octan-Dihydrochlorid [J. Med. Chem. 17, 481 (1974)],

3-Methyl-3,8-diazabicyclo[3.2.1]octan-Dihydrochlorid [J. Med. Chem. 17, 481 (1974)],

2,5-Diazabicyclo[2.2.1]heptan-Dihydrochlorid [J. Org. Chem. 31, 1059 (1966)],

2-Methyl-2,5-diazabicyclo[2.2.1]heptan-Dihydrochlorid [J. Org. Chem. 31, 1059 (1966); J. Med. Chem. 17 481 (1974)],

2-Benzyl-2,5-diazabicyclo[2.2.1]heptan-Dihydrochlorid [J. Org. Chem. 31, 1059 (1966)],

5-Methyl-1,4-diazabicyclo[3.2.1]octan,

8-Ethyl-3,8-diazabicyclo[3.2.1]octan,

8-Benzyl-3,8-diazabicyclo[3.2.1]octan,

8-(4-Nitrobenzyl)-3,8-diazabicyclo[3.2.1]octan,

8-(4-Aminobenzyl)-3,8-diazabicyclo[3.2.1]octan,

3-Ethyl-3,8-diazabicyclo[3.2.1]octan,

3-Benzyl-3,8-diazabicyclo[3.2.1] octan,

3-(4-Aminobenzyl)-3,8-diazabicyclo[3.2.1]octan,

2,5-Diazabicyclo[2.2.2]octan-Dihydrochlorid [J. Med. Chem. 17, 481 (1974)],

2-Methyl-2,5-diazabicyclo[2.2.2]octan-Dihydrochlorid [J. Med. Chem. 17, 481 (1974)],

2-Ethyl-2,5-diazabicyclo[2.2.2]octan,

2-Benzyl-2,5-diazabicyclo[2.2.2]octan,

2-(4-Aminobenzyl)-2,5-diazabicylo[2.2.2]octan,

8-Propyl-3,8-diazabicyclo[3.2.1]octan,

3-Isopropyl-3,8-diazabicyclo[3.2.1]octan,

2-Butyl-2,5-diazabicyclo[2.2.1]heptan,

2-Isobutyl-2,5-diazabicyclo[2.2.2]octan,

8-(2-Hydroxyethyl)-3,8-diazabicyclo[3.2.1]octan,

3-(2-Hydroxyethyl)-3,8-diazabicyclo[3.2.1]octan,

2-(2-Hydroxyethyl)-2,5-diazabicyclo[2.2.1]heptan,

2-(2-Hydroxyethyl)-2,5-diazabicyclo[2.2.2]octan,

1,4-Diazabicyclo[3.1.1]heptan,

3,9-Diazabicyclo[3.3.1]nonan,

7-Hydroxy-3,9-diazabicyclo[3.3.1]nonan [Chem. Heterocyclic Compounds USSR 1, 195 (1965)],

7-Hydroxy-9-methyl-3,9-diazabicyclo[3.3.1]nonan,

3-Oxa-7,9-diazabicyclo[3.3.1]nonan,

3-Oxa-9-azabicyclo[3.3.1]nonan,

2,5-Diazabicyclo[3.1.1]heptan,

1,4-Diazabicyclo[3.3.1]nonan,

2,5,9-Triazabicyclo[3.2.2]nonan,

6-Thia-3,8-diazabicyclo[3.2.1]octan.

Die Ausgangsverbindungen der Formel IV sind unbekannt. Sie entsprechen den erfindungsgemäßen Verbindungen der Formel I, in welcher der Rest $R^4$ für Wasserstoff steht.

Die Ausgangsverbindungen der Formel V sind bekannt. Als Beispiele seien genannt:

Methyljodid, Methylbromid, Methylchlorid, Ethyljodid, Ethylbromid, Benzylbromid, Benzylchlorid, 4-Nitrobenzylbromid, 2-Chlorethanol, 2-Bromethanol, 2-Jodethanol, 3-Brompropanol, 4-Jodbutanol, Chloraceton, 1-Chlor-2-butanon, 1-Brom-3-butanon, Allylbromid, Propargylbromid. Auch Methylvinylketon ist bekannt.

Die Umsetzung von (II) mit (III) gemäß Methode A, bei der die Diazabicycloalkane (III) auch in Form ihrer Hydrochloride eingesetzt werden können, wird vorzugsweise in einem Verdünnungsmittel wie Dimethylsulfoxid, N,N-Dimethylformamid, Hexamethyl-phosphorsäuretrisamid, Sulfolan, Acetonitril, Wasser, einem Alkohol wie Methanol, Ethanol, n-Propanol, Isopropanol, Glykolmonomethylether oder Pyridin vorgenommen. Ebenso können Gemische dieser Verdünnungsmittel verwendet werden.

Als Säurebinder können alle üblichen anorganischen und organischen Säurebindungsmittel verwendet werden. Hierzu gehören vorzugsweise die Alkalihydroxide, Alkalicarbonate, organische Amine und Amidine. Als besonders geeignet seien im einzelnen genannt: Triethylamin, 1,4-Diaza-bicyclo-[2.2.2]octan (DABCO), 1,8-Diazabicyclo[5,4,0]undec-7-en (DBU) oder überschüssiges Amin (III).

Die Reaktionstemperaturen können in einem größeren Bereich variiert werden. Im allgemeinen arbeitet man zwischen etwa 20 und 200°C, vorzugsweise zwischen 80 und 180°C.

Die Umsetzung kann bei Normaldruck, aber auch bei erhöhtem Druck durchgeführt werden. Im allgemeinen arbeitet man bei Drucken zwischen etwa 1 und 100 bar, vorzugsweise zwischen 1 und 10 bar.

Bei der Durchführung des erfindungsgemäßen Verfahrens setzt man auf 1 Mol der Carbonsäure (II) 1 bis 15 Mol, vorzugsweise 1 bis 6 Mol der Verbindung (III) ein.

Freie Hydroxygruppen können während der Umsetzung durch eine geeignete Hydroxyschutzgruppe, zum Beispiel durch den Tetrahydropyranylrest, geschützt und nach Beendigung der Reaktion wieder freigesetzt werden.

Die Umsetzung von (IV) mit (V) gemäß Methode B wird vorzugsweise in einem Verdünnungsmittel wie Dimethylsulfoxid, Dioxan, N,N-Dimethylformamid, Hexamethyl-phosphorsäuretrisamid, Sulfolan, Wasser, einem Alkohol wie Methanol, Ethanol, n-Propanol, Isopropanol, Glykolmonomethylether oder Pyridin vorgenommen. Ebenso können Gemische dieser Verdünnungsmittel verwendet werden.

Als Säurebinder können alle üblichen anorganischen und organischen Säurebindemittel verwendet werden. Hierzu gehören vorzugsweise die Alkalihydroxide, Alkalicarbonate, organische Amine und Amidine. Als besonders geeignet seien im einzelnen genannt: Triethylamin, 1,4-Diazabicyclo-[2.2.2]octan (DABCO) oder 1,8-Diazabicyclo[5,4,0]undec-7-en (DBU).

Zur Beschleunigung der Umsetzung können Phasentransferkatalysatoren wie Tetrabutylammoniumbromid, Tetrabutylammioniumchlorid oder Benzyl-triethyl-ammoniumchlorid zugegeben werden.

Die Reaktionstemperaturen können in einem größeren Bereich variiert werden. Im allgemeinen arbeitet man zwischen etwa 20 und etwa 180° C, vorzugsweise zwischen 40 und 110° C.

Die Umsetzung kann bei Normaldruck, aber auch bei erhöhtem Druck durchgeführt werden. Im allgemeinen arbeitet man bei Drucken zwischen etwa 1 und etwa 100 bar, vorzugsweise zwischen 1 und 10 bar.

Bei der Durchführung des erfindungsgemäßen Verfahrens gemäß Methode B setzt man auf 1 Mol der Verbindung (IV) 1 bis 4 Mol, vorzugsweise 1 bis 1,5 Mol der Verbindung (V) ein.

Die Umsetzung von (IV) mit Methylvinylketon (Methode C) wird vorzugsweise in einem Verdünnungsmittel wie Dioxan, Dimethylsulfoxid, N,N-Dimethylformamid, Methanol, Ethanol, Isopropanol, n-Propanol, Glykolmonomethylether oder auch in Gemischen dieser Verdünnungsmittel durchgeführt.

Die Reaktionstemperaturen können in einem größeren Bereich variiert werden. Im allgemeinen arbeitet man zwischen etwa 20° C und etwa 150° C, vorzugsweise zwischen 50° C und 100° C.

Die Umsetzung kann bei Normaldurck, aber auch bei erhöhtem Druck durchgeführt werden. Im allgemeinen arbeitet man bei Drucken zwischen etwa 1 und etwa 100 bar, vorzugsweise zwischen 1 und 10 bar.

Bei der Durchführung des erfindungsgemäßen Verfahrens setzt man auf 1 Mol der Verbindung (IV) 1 bis 5 Mol, vorzugsweise 1 bis 2 Mol Methylvinylketon ein.

Zur Herstellung der erfindungsgemäßen Ester wird die zugrundeliegende Carbonsäure vorzugsweise in überschüssigem Alkohol in Gegenwart von starken Säuren, wie Schwefelsäure, wasserfreiem Chlorwasserstoff, Methansulfonsäure, p-Toluolsulfonsäure oder sauren Ionenaustauschern, bei Temperaturen von etwa 20° bis 200° C, vorzugsweise etwa 60° bis 120° C umgesetzt. Das entstehende Reaktionswasser kann auch durch azeotrope Destillation mit Chloroform, Tetrachlormethan, Benzol oder Toluol entfernt werden.

Die als Prodrug verwendeten (5-Methyl-2-oxo-1,3-dioxol-4-yl-methyl)-ester werden durch Umsetzung eines Alkalisalzes der zugrundeliegenden Carbonsäure mit 4-Brommethyl- oder 4-Chlormethyl-5-methyl-1,3-dioxol-2-on in einem Lösungsmittel wie Dimethylformamid, Dimethylacetamid, Dimethylsulfoxid oder Tetramethylharnstoff bei Temperaturen von etwa 0° bis 100° C, vorzugsweise 0° bis 50° C, erhalten.

Die Einführung eines Aminobenzylrestes $R^4$ erfolgt durch Reduktion eines bereits im Wirkstoff der Formel I eingeführten Nitrobenzylrestes durch katalytisch angeregten Wasserstoff oder chemisch durch Reduktion mit Eisen oder Zink.

Die Herstellung der Säureadditionssalze der erfindungsgemäßen Verbindungen erfolgt in üblicher Weise zum Beispiel durch Lösen des Betains in überschüssiger wäßriger Säure und Ausfällen des Salzes mit einem mit Wasser mischbaren organischen Lösungsmittel wie Methanol, Ethanol, Aceton, Acetonitril. Man kann auch äquivalente Mengen Betain und Säure in Wasser oder einem Alkohol wie Glykolmonomethylether erhitzen und anschließend bis zur Trockne eindampfen oder das ausgefallene Salz absaugen. Als pharmazeutisch verwendbare Salze sind beispielsweise die Salze von Salzsäure, Schwefelsäure, Essigsäure, Glykolsäure, Milchsäure, Bernsteinsäure, Zitronensäure, Weinsäure, Methansulfonsäure, Galacturonsäure, Gluconsäure, Embonsäure, Glutaminsäure oder Asparaginsäure zu verstehen.

Die Alkali- oder Erdalkalisalze der erfindungsgemäßen Carbonsäuren werden beispielsweise durch Lösen des Betains in unterschüssiger Alkali- oder Erdalkalilauge, Filtration von ungelöstem Betain und Eindampfen des Filtrats bis zur Trockne erhalten. Pharmazeutisch geeignet sind Natrium-, Kalium- oder Calciumsalze. Durch Umsetzung eines Alkali- oder Erdalkalisalzes mit einem geeigneten Silbersalz wie Silbernitrat werden die entsprechenden Silbersalze erhalten.

Die erfindungsgemäßen Wirkstoffe können sowohl als Racemate als auch als enantiomerenreine Verbindungen vorliegen.

Außer den in den Beispielen aufgeführten Verbindungen seien als neue Wirkstoffe im einzelnen genannt:

6-Chlor-1-cyclopropyl-7-(1,4-diazabicyclo[3.2.1]-oct-4-yl)-1,4-dihydro-4-oxo-3-chinolincarbonsäure,

1-Cyclopropyl-7-(1,4-diazabicyclo[3.2.1]-oct-4-yl)-6-fluor-1,4-dihydro-8-methyl-4-oxo-3-chinolincarbonsäure,

7-(1,4-Diazabicyclo[3.2.1]oct-4-yl)-6-fluor-1-(2-fluorethyl)-1,4-dihydro-4-oxo-3-chinolincarbonsäure,

7-(1,4-Diazabicyclo[3.2.1]oct-4-yl)-6-fluor-1-(4-fluorphenyl)-1,4-dihydro-4-oxo-3-chinolincarbonsäure,

1-Cyclopropyl-7-(1,4-diazabicyclo[3.2.1]oct-4-yl)-6-fluor-1,4-dihydro-4-oxo-1,8-naphthyridin-3-carbonsäure,

7-(1,4-Diazabicyclo[3.2.1]oct-4-yl)-1-ethyl-6-fluor-1,4-dihydro-4-oxo-1,8-naphthyridin-3-carbonsäure,

7-(1,4-Diazabicyclo[3.2.1]oct-4-yl)-1-ethyl-6-fluor-1,4-dihydro-4-oxo-3-chinolincarbonsäure,

7-(1,4-Diazabicyclo[3.2.1]oct-4-yl)-9-fluor-6,7-dihydro-5-methyl-1-oxo-1H,5H-benzo[i,j]chinolin-2-carbonsäure,

7-(1,4-Diazabicyclo[3.2.1]oct-4-yl)-6-fluor-1-(4-fluorphenyl)-1,4-dihydro-4-oxo-1,8-naphthyridin-3-carbonsäure,

1-Cyclopropyl-7-(1,4-diazabicyclo[3.2.1]oct-4-yl)-6-fluor-1,4-dihydro-4-oxo-3-chinolincarbonsäureethylester,

1-Cyclopropyl-7-(1,4-diazabicyclo[3.2.1]oct-4-yl)-6-fluor-1,4-dihydro-4-oxo-3-chinolincarbonsäure-(5-methyl-2-oxo-1,3-dioxol-4-yl-methyl)-ester,

1-Cyclopropyl-7-(1,4-diazabicyclo[3.1.1]hept-4-yl)-6-fluor-1,4-dihydro-4-oxo-3-chinolincarbonsäure,

1-Cyclopropyl-7-(1,4-diazabicyclo[3.1.1]hept-4-yl)-6,8-difluor-1,4-dihydro-4-oxo-3-chinolincarbonsäure,

8-Chlor-1-cyclopropyl-7-(1,4-diazabicyclo[3.1.1]hept-4-yl)-6-fluor-1,4-dihydro-4-oxo-3-chinolincarbonsäure,

1-Cyclopropyl-7-(1,4-diazabicyclo[3.1.1]hept-4-yl)-6-fluor-1,4-dihydro-8-methyl-4-oxo-3-chinolincarbonsäure,

1-Cyclopropyl-7-(1,4-diazabicyclo[3.1.1]hept-4-yl)-6-fluor-1,4-dihydro-4-oxo-1,8-naphthyridin-3-carbonsäure,

1-Cyclopropyl-7-(1,4-diazabicyclo[3.1.1]hept-4-yl)-6-fluor-1,4-dihydro-4-oxo-3-chinolincarbonsäure-methylester,

1-Cyclopropyl-6,8-difluor-1,4-dihydro-7-(8-methyl-3,8-diazabicyclo[3.2.1]oct-3-yl)-4-oxo-3-chinolincarbonsäure,

8-Chlor-1-cyclopropyl-6-fluor-1,4-dihydro-7-(8-methyl-3,8-diazabicyclo[3.2.1]oct-3-yl)-4-oxo-3-chinolincarbonsäure,

1-Cyclopropyl-6-fluor-1,4-dihydro-7-(8-methyl-3,8-diazabicyclo[3.2.1]oct-3-yl)-4-oxo-1,8-naphthyridin-3-carbonsäure,

1-Cyclopropyl-6-fluor-1,4-dihydro-7-(3-methyl-3,8-diazabicyclo[3.2.1]oct-8-yl)-4-oxo-3-chinolincarbonsäure,

1-Cyclopropyl-6,8-difluor-1,4-dihydro-7-(3-methyl-3,8-diazabicyclo[3.2.1]oct-8-yl)-4-oxo-3-chinolincarbonsäure,

8-Chlor-1-cyclopropyl-6-fluor-1,4-dihydro-7-(3-methyl-3,8-diazabicyclo[3.2.1]oct-8-yl)-4-oxo-3-chinolincarbonsäure,

1-Cyclopropyl-7-(2,5-diazabicyclo[2.2.1]hept-2-yl)-6-fluor-1,4-dihydro-4-oxo-3-chinolincarbonsäure,

1-Cyclopropyl-7-(2,5-diazabicyclo[2.2.1]hept-2-yl)-6,8-difluor-1,4-dihydro-4-oxo-3-chinolincarbonsäure,

8-Chlor-1-cyclopropyl-7-(2,5-diazabicyclo[2.2.1]-hept-2-yl)-7-fluor-1,4-dihydro-4-oxo-3-chinolincarbonsäure,

1-Cyclopropyl-7-(2,5-diazabicyclo[2.2.1]hept-2-yl)-6-fluor-1,4-dihydro-4-oxo-1,8-naphthyridin-3-carbonsäure,

1-Cyclopropyl-6-fluor-1,4-dihydro-7-[5-(2-hydroxyethyl)-2,5-diazabicyclo[2.2.1]hept-2-yl]-4-oxo-3-chinolincarbonsäure,

1-Cyclopropyl-6-fluor-1,4-dihydro-4-oxo-7-[5-(2-oxopropyl)-2,5-diazabicyclo[2.2.1]hept-2-yl]-3-chinolincarbonsäure,

1-Cyclopropyl-6-fluor-1,4-dihydro-7-(5-methyl-2,5-diazabicyclo[2.2.1]hept-2-yl)-4-oxo-3-chinolincarbonsäure,

1-Cyclopropyl-6,8-difluor-1,4-dihydro-7-(5-methyl-2,5-diazabicyclo[2.2.1]hept-2-yl)-4-oxo-3-chinolincarbonsäure,

8-Chlor-1-cyclopropyl-6-fluor-1,4-dihydro-7-(5-methyl-2,5-diazabicyclo[2.2.1]hept-2-yl)-4-oxo-3-chinolincarbonsäure,

1-Cyclopropyl-7-(8-ethyl-3,8-diazabicyclo[3.2.1]oct-3-yl)-6-fluor-1,4-dihydro-4-oxo-3-chinolincarbonsäure,

1-Cyclopropyl-6-fluor-1,4-dihydro-4-oxo-7-[8-(3-oxobutyl)-3,8-diazabicyclo[3.2.1]oct-3-yl]-3-chinolincarbonsäure,

7-[8-(4-Aminobenzyl)-3,8-diazabicyclo[3.2.1]oct-3-yl]-1-cyclopropyl-6-fluor-1,4-dihydro-4-oxo-3-chinolincarbonsäure,

1-Cyclopropyl-7-(2,5-diazabicyclo[2.2.2]oct-2-yl)-6-fluor-1,4-dihydro-4-oxo-3-chinolincarbonsäure,

1-Cyclopropyl-7-(2,5-diazabicyclo[2.2.2]oct-2-yl)-6,8-difluor-1,4-dihydro-4-oxo-3-chinolincarbonsäure,

8-Chlor-1-cyclopropyl-7-(2,5-diazabicyclo[2.2.2]oct-2-yl)-7-fluor-1,4-dihydro-4-oxo-3-chinolincarbonsäure,

1-Cyclopropyl-7-(2,5-diazabicyclo[2.2.2]oct-2-yl)-6-fluor-1,4-dihydro-4-oxo-1,8-naphthyridin-3-carbonsäure,

1-Cyclopropyl-6-fluor-1,4-dihydro-7-(5-methyl-2,5-diazabicyclo[2.2.2]oct-2-yl)-4-oxo-3-chinolincarbonsäure,

1-Cyclopropyl-6,8-difluor-1,4-dihydro-7-(5-methyl-2,5-diazabicyclo[2.2.2]oct-2-yl)-4-oxo-3-chinolincarbonsäure,

8-Chlor-1-cyclopropyl-6-fluor-1,4-dihydro-7-(5-methyl-2,5-diazabicyclo[2.2.2]oct-2-yl)-4-oxo-3-chinolincarbonsäure,

1-Cyclopropyl-7-(3,9-diazabicyclo[3.3.1]non-3-yl)-6-fluor-1,4-dihydro-4-oxo-3-chinolincarbonsäure,

1-Cyclopropyl-6-fluor-1,4-dihydro-7-(7-hydroxy-3,9-diazabicyclo[3.3.1]non-3-yl)-4-oxo-3-chinolincarbonsäure,

1-Cyclopropyl-6-fluor-1,4-dihydro-7-(3-oxa-7,9-diazabicyclo[3.3.1]non-7-yl)-4-oxo-3-chinolincarbonsäure,

7-(5-Allyl-2,5-diazabicyclo[2.2.1]hept-2-yl)-1-cyclopropyl-6-fluor-1,4-dihydro-4-oxo-3-chinolincarbonsäure,

1-Cyclopropyl-6-fluor-1,4-dihydro-4-oxo-7-(5-propargyl-2,5-diazabicyclo[2.2.2]oct-2-yl)-3-chinolincarbonsäure.

Beispiel für eine erfindungsgemäße Tablette

Jede Tablette enthält:

| | |
|---|---|
| Verbindung des Beispiels 1 | 583,0 mg |
| Mikrokristalline Cellulose | 55,0 mg |
| Maisstärke | 72,0 mg |
| Poly-(1-vinyl-2-pyrrolidon) unlöslich | 30,0 mg |
| Hochdisperses Siliciumdioxid | 5,0 mg |
| Magnesiumstearat | 5,0 mg |
| | 750,0 mg |

Die Lackhülle enthält:

| | |
|---|---|
| Poly-(O-hydroxypropyl-O-methyl)-cellulose 15 cp | 6,0 mg |
| Macrogol 4000 rec. INN Polyethylenglykole (DAB) | 2,0 mg |
| Titan-(IV)-oxid | 2,0 mg |
| | 10,0 mg |

Die erfindungsgemäßen Verbindungen zeigen bei geringer Toxizität ein breites antibakterielles Spektrum gegen gram-positive und gram-negative Keime, insbesondere gegen Enterobakteriaceen; vor allem auch gegen solche, die resistent sind gegen verschiedene Antibiotika, wie z.B. Penicilline, Cephalosporine, Aminoglykoside, Sulfonamide, Tetracycline.

Diese wertvollen Eigenschaften ermöglichen ihre Verwendung als chemotherapeutische Wirkstoffe in der Medizin sowie als Stoffe zur Konservierung von anorganischen und organischen Materialien, insbesondere von organischen Materialien aller Art, z.B. Polymeren, Schmiermitteln, Farben, Fasern, Leder, Papier und Holz, von Lebensmitteln und von Wasser.

Die erfindungsgemäßen Verbindungen sind gegen ein sehr breites Spektrum von Mikroorganismen wirksam. Mit ihrer Hilfe können gram-negative und gram-positive Bakterien und bakterienähnliche Mikroorganismen bekämpft sowie die durch diese Erreger hervorgerufenen Erkrankungen verhindert, gebessert und/oder geheilt werden.

Besonders wirksam sind die erfindungsgemäßen Verbindungen gegen Bakterien und bakterienähnliche Mikroorganismen. Sie sind daher besonders gut zur Prophylaxe und Chemotherapie von lokalen und systemischen Infektionen in der Human- und Tiermedizin geeignet, die durch diese Erreger hervorgerufen werden.

Beispielsweise können lokale und/oder systemische Erkrankungen behandelt und/oder verhindert werden, die durch die

folgenden Erreger oder durch Mischungen der folgenden Erreger verursacht werden: Gram-positive Kokken, z.B. Staphylokokken (Staph. aureus, Staph. epidermidis) und Streptokokken (Strept. agalactiae, Strept. faecalis, Strept. pneumoniae, Strept. pyogenes); gram-negative Kokken (Neisseria gonorrhoeae) sowie gram-negative Stäbchen wie Enterobakteriaceen, z.B. Escherichia coli, Hämophilus influenzae, Citrobacter (Citrob. freundii, Citrob. divernis), Salmonella und Shigella; ferner Klebsiellen (Klebs. pneumoniae, Klebs. oxytoca), Enterobacter (Ent. aerogenes, Ent. agglomerans), Hafnia, Serratia (Serr. marcescens), Proteus (Pr. mirabilis, Pr. rettgeri, Pr. vulgaris), Providencia, Yersinia, sowie die Gattung Acinetobacter. Darüber hinaus umfaßt das antibakterielle Spektrum die Gattung Pseudomonas (Ps. aeruginosa, Ps. maltophilia) sowie strikt anaerobe Bakterien wie z.B. Bacteroides fragilis, Vertreter der Gattung Peptococcus, Peptostreptococcus sowie die Gattung Clostridium; ferner Mykoplasmen (M. pneumoniae, M. hominis, M. urealyticum) sowie Mykobakterien, z.B. Mycobacterium tuberculosis.

Die obige Aufzählung von Erregern ist lediglich beispielhaft und keineswegs beschränkend aufzufassen. Als Krankheiten, die durch die genannten Erreger oder Mischinfektionen verursacht und durch die erfindungsgemäßen Verbindungen verhindert, gebessert oder geheilt werden können, seien beispielsweise genannt:

Infektionskrankheiten beim Menschen wie zum Beispiel Otitis, Pharyngitis, Pneumonie, Peritonitis, Pyelonephritis, Cystitis, Endocarditis, Systeminfektionen, Bronchitis (akut, chronisch), septische Infektionen, Erkrankungen der oberen Luftwege, diffuse Panbronchiolitis, pulmonäres Emphysem, Dysenterie, Enteritis, Leberabszesse, Urethritis, Prostatitis, Epididymitis, gastrointestinale Infektionen, Knochen- und Gelenkinfektionen, zystische Fibrose, Hautinfektionen, postoperative Wundinfektionen, Abszesse, Phlegmone, Wundinfektionen, infizierte Verbrennungen, Brandwunden, Infektionen im Mundbereich, Infektionen nach Zahnoperationen, Osteomyelitis, septische Arthritis, Cholecystitis, Peritonitis mit Appendicitis, Cholangitis, intraabdominale Abszesse, Pankreatitis, Sinusitis, Mastoiditis, Mastitis, Tonsillitis, Typhus, Meningitis und Infektionen des Nervensystems, Salpingitis, Endometritis, Genital-Infektionen, Pelveoperitonitis und Augeninfektionen.

Außer beim Menschen können bakterielle Infektionen auch bei anderen Spezies behandelt werden. Beispielhaft seien genannt:
Schwein: Coli-diarrhoe, Enterotoxämie, Sepsis, Dysenterie, Salmonellose, Metritis-Mastitis-Agalaktiae-Syndrom, Mastitis;
Wiederkäuer (Rind, Schaf, Ziege): Diarrhoe, Sepsis, Bronchopneumonie, Salmonellose, Pasteurellose, Mykoplasmose, Genitalinfektionen;
Pferd: Bronchopneumonien, Fohlenlähme, puerperale und postpuerperale Infektionen, Salmonellose;
Hund und Katze: Bronchopneumonie, Diarrhoe, Dermatitis, Otitis, Harnwegsinfekte, Prostatitis;

Geflügel (Huhn, Pute, Wachtel, Taube, Ziervögel und andere): Mycoplasmose, E. coli-Infektionen, chronische Luftwegserkrankungen, Salmonellose, Pasteurellose, Psittakose.

Ebenso können bakterielle Erkrankungen bei der Aufzucht und Haltung von Nutz- und Zierfischen behandelt werden, wobei sich das antibakterielle Spektrum über die vorher genannten Erreger hinaus auf weitere Erreger wie zum Beispiel Pasteurella, Brucella, Campylobacter, Listeria, Erysipelothrix, Corynebakterien, Borellia, Treponema, Nocardia, Rikettsien, Yersinia, erweitert.

Zur vorliegenden Erfindung gehören pharmazeutische Zubereitungen, die neben nicht-toxischen, inerten pharmazeutisch geeigneten Trägerstoffen eine oder mehrere erfindungsgemäße Verbindungen enthalten oder die aus einem oder mehreren erfindungsgemäßen Wirkstoffen bestehen, sowie Verfahren zur Herstellung dieser Zubereitungen.

Zur vorliegenden Erfindung gehören auch pharmazeutische Zubereitungen in Dosierungseinheiten. Dies bedeutet, daß die Zubereitung in Form einzelner Teile, z.B. Tabletten, Dragees, Kapseln, Pillen, Suppositorien und Ampullen vorliegen, deren Wirkstoffgehalt einem Bruchteil oder einem Vielfachen einer Einzeldosis entsprechen. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht-toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Tabletten, Dragees, Kapseln, Pillen und Granulate können den oder die Wirkstoffe neben den üblichen Trägerstoffen enthalten, wie (a) Füll- und Streckmittel, z.B. Stärken, Milchzucker, Rohrzucker, Glukose, Mannit und Kieselsäure, (b) Bindemittel, z.B. Carboxymethylcellulose, Alginate, Gelatine, Polyvinylpyrrolidon, (c) Feuchthaltemittel, z.B. Glycerin, (d) Sprengmittel, z.B. Agar-Agar, Calciumcarbonat und Natriumcarbonat, (e) Lösungsverzögerer, z.B. Paraffin und (f) Resorptionsbeschleuniger, z.B. quarternäre Ammoniumverbindungen, (g) Netzmittel, z.B. Cetylalkohol, Glycerinmonostearat, (h) Adsorptionsmittel, z.B. Kaolin und Bentonit und (i) Gleitmittel z.B. Talkum, Calcium- und Magnesiumstearat und feste Polyethylenglykole oder Gemische der unter (a) bis (i) aufgeführten Stoffe.

Die Tabletten, Dragees, Kapseln, Pillen und Granulate können mit den üblichen, gegebenenfalls Opakisierungsmitteln enthaltenden, Überzügen und Hüllen versehen sein und auch so zusammengesetzt sein, daß sie den oder die Wirkstoffe nur oder bevorzugt in einem bestimmten Teil des Intesti-

0230274

naltraktes gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z.B. Polyethylenglykole, Fette, z.B. Kakaofett und höhere Ester (z.B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten, z.B. tierische und pflanzliche Fette, Wachse, Paraffine, Stärke, Tragant, Cellulosederivate, Polyethylenglykole, Silikone, Bentonite, Kieselsäure, Talkum und Zinkoxid oder Gemische dieser Stoffe.

Puder und Sprays können neben dem oder den Wirkstoffen die üblichen Trägerstoffe enthalten, z.B. Milchzucker, Talkum, Kieselsäure, Aluminiumhydroxid, Calciumsilikat und Polyamidpulver oder Gemische dieser Stoffe. Sprays können zusätzlich die üblichen Treibmittel, z.B. Chlorfluorkohlenwasserstoffe, enthalten.

Lösungen und Emulsionen können neben dem oder den Wirkstoffen die üblichen Trägerstoffe wie Lösungsmittel, Lösungsvermittler und Emulgatoren, z.B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydofurfurylalkohol, Polyethylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z.B. Wasser, Ethylalkohol, Propylenglykol, Suspendiermittel, z.B. ethoxylierte Isostearylalkohole, Polyoxyethylensorbit- und Sorbitan-Ester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmacksverbesserte Zusätze, z.B. Pfefferminzöl und Eukalyptusöl und Süßmittel, z.B. Saccharin, enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-%, der Gesamtmischung vorhanden sein.

Die oben aufgeführten pharmazeutischen Zubereitungen können außer den erfindungsgemäßen Verbindungen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Die genannten Zubereitungen können bei Mensch und Tier entweder oral, rektal, parenteral (intravenös, intramuskulär, subkutan), intracisternal, intravaginal, intraperitoneal, lokal (Puder, Salbe, Tropfen) und zur Therapie von Infektionen in Hohlräumen, Körperhöhlen angewendet werden. Als geeignete Zubereitungen kommen Injektionlösungen, Lösungen und Suspensionen für die orale Therapie, Gele, Aufgußformulierungen, Emulsionen, Salben oder Tropfen in Frage. Zur lokalen Therapie können ophtalmologische und dermatologische Formulierungen, Silber- und andere Salze, Ohrentropfen, Augensalben, Puder oder Lösungen verwendet werden. Bei Tieren kann die Aufnahme auch über das Futter oder Trinkwasser in geeigneten Formulierungen erfolgen. Ferner können Gele, Pulver, Puder, Tabletten, Retard-Tabletten, Premixe, Konzentrate, Granulate, Pellets, Boli, Kapseln, Aerosole, Sprays, Inhalate bei Mensch und Tier angewendet werden. Ferner können die erfindungsgemäßen Verbindungen in andere Trägermaterialien wie zum Beispiel Kunststoffe, (Kunststoffketten zur lokalen Therapie), Kollagen oder Knochenzement eingearbeitet werden.

Im allgemeinen hat es sich sowohl in der Human- als auch in der Veterinärmedizin als vorteilhaft erwiesen, den oder die erfindungsgemäßen Wirkstoffe in Gesamtmengen von etwa 0,5 bis etwa 500, vorzugsweise 5 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die erfindungsgemäßen Wirkstoffe vorzugsweise in Mengen von etwa 1 bis etwa 80, insbesondere 3 bis 30 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt.

So kann es in einigen Fällen ausreichend sein, mit weniger als der obengenannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Die neuen Verbindungen können in den üblichen Konzentrationen und Zubereitungen zusammen mit dem Futter bzw. mit Futterzubereitungen oder mit dem Trinkwasser gegeben werden. Dadurch kann eine Infektion durch gram-negative oder gram-positive Bakterien verhindert, gebessert und/oder geheilt werden und dadurch eine Förderung des Wachstums und eine Verbesserung der Verwertung des Futters erreicht werden.

In der nachstehenden Tabelle sind einige MHK-Werte im Vergleich zu 1-Cyclopropyl-6-fluor-1,4-dihydro-4-oxo-7-(1-piperazinyl)-3-chinolincarbonsäure (Ciprofloxacin) angegeben:

| Beispiel / Stamm | MHK (mcg/ml) *) 1 | 3 | 12 | 4 | Ciprofloxacin |
|---|---|---|---|---|---|
| E.coli 455/7 | 2 | 0,5 | 4 | 0,5 | 2 |
| Providenicia 12052 | — | 8 | 16 | 8 | 32 |
| Staph. aur. FK 422 | 0,25 | 0,06 | 0,125 | 0,125 | 0,5 |
| Staph. aur. 1756 | 0,25 | 0,06 | 0,125 | 0,06 | 0,5 |
| Staph. aur. 133 | 0,125 | 0,06 | 0,125 | 0,06 | 0,5 |
| Streptoc. faecal. 27101 | 0,25 | 0,06 | 0,125 | 0,125 | 0,5 |
| Streptoc. faecal. 9790 | 0,25 | 0,06 | 0,125 | 0,25 | 0,25 |
| Pseudomonas aerug. W. | 0,5 | 0,125 | 0,5 | 0,5 | 0,5 |

*) Agardilutionstest/Isosensitestagar

Die folgenden Beispiele erläutern die Erfindung:

Beispiel 1

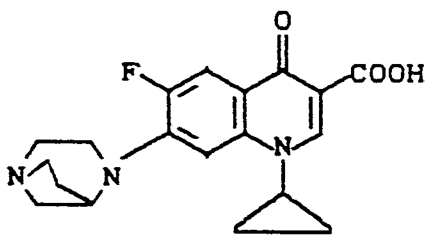

·HCl

8,1 g (30 mmol) 1-Cyclopropyl-6,7-difluor-1,4-dihydro-4-oxo-3-chinolincarbonsäure in 60 ml Acetonitril und 90 ml Dimethylformamid werden mit 3,4 g (30 mmol) 1,4-Diazabicyclo[3.2.1]octan und 6,6 g (59 mmol) 1,4-Diazabicyclo-[2.2.2]octan versetzt und 6 Stunden unter Rückfluß erhitzt. Nach dem Abkühlen wird die Mischung eingeengt, der Rückstand mit Wasser verrührt und das ungelöste Betain abgesaugt (Schmelzpunkt ab etwa 258°C unter Zersetzung). Dieses wird in 5 ml halbkonzentrierter Salzsäure heiß gelöst, filtriert, mit 40 ml Ethanol versetzt und mit Eis gekühlt. Das ausgefallene Hydrochlorid wird abgesaugt, mit Ethanol gewaschen und getrocknet. Man erhält 5,3 g (45 % der Theorie) 1-Cyclopropyl-7-(1,4-diazabicyclo[3.2.1]oct-4-yl)-6-fluor-1,4-dihydro-4-oxo-3-chinolincarbonsäure-Hydrochlorid vom Schmelzpunkt 322°C (unter Zersetzung).

Beispiel 2

2 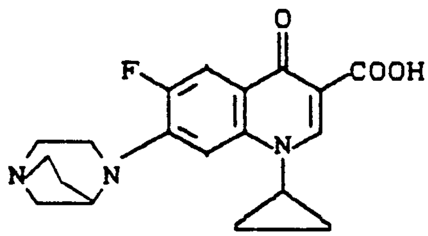 x 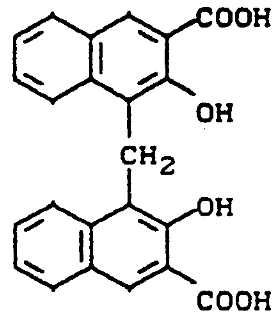

3,3 g (9,2 mmol) 1-Cyclopropyl-7-(1,4-diazabicyclo[3.2.1]-octan-4-yl)-6-fluor-1,4-dihydro-4-oxo-3-chinolincarbonsäure (Betain aus Beispiel 1) und 1,8 g (4,6 mmol) Embonsäure werden in 35 ml Glykolmonomethylether 4 Stunden unter Rückfluß erhitzt. Die Suspension wird abgekühlt, das Kristallisat abgesaugt, mit Ethanol gewaschen und bei 120° C im Vakuum getrocknet. Man erhält 4,7 g 1-Cyclopropyl-7-(1,4-diazabicyclo[3.2.1]oct-4-yl)-6-fluor-1,4-dihydro-4-oxo-3-chinolincarbonsäure-Hemiembonat vom Schmelzpunkt ab 271° C (unter Zersetzung).

Beispiel 3

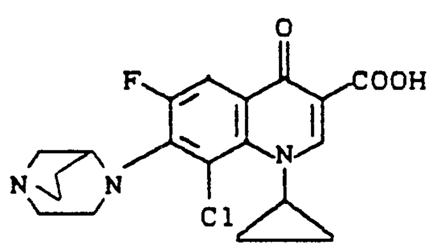 · HCl · $H_2O$

Man setzt 8-Chlor-1-cyclopropyl-6,7-difluor-1,4-dihydro-4-oxo-3-chinolincarbonsäure analog Beispiel 1 um und erhält 8-Chlor-1-cyclopropyl-7-(1,4-diazabicyclo[3.2.1]oct-4-yl)-6-fluor-1,4-dihydro-4-oxo-3-chinolincarbonsäure-Hydrochlorid-Hydrat vom Schmelzpunkt 310° C (unter Zersetzung).

$C_{19}H_{19}ClFN_3O_3 \cdot HCl \cdot H_2O$ (446,3)

| | | | | |
|---|---|---|---|---|
| Berechnet = | C 51,1 | H 4,96 | N 9,41 | Cl 15,9 |
| Gefunden = | C 51,2 | H 4,6 | N 9,5 | Cl 15,6 |

Beispiel 4

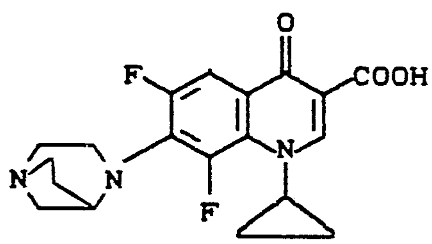

x HCl

Man setzt 1-Cyclopropyl-6,7,8-trifluor-1,4-dihydro-4-oxo-3-chinolincarbonsäure analog Beispiel 1 um und erhält 1-Cyclopropyl-7-(1,4-diazabicyclo[3.2.1]oct-4-yl)-6,8-difluor-1,4-dihydro-4-oxo-3-chinolincarbonsäure vom Schmelzpunkt 275-282° C (unter Zersetzung) und daraus das Hydrochlorid vom Schmelzpunkt >310° C unter Zersetzung (schon ab etwa 260° C wird die Substanz langsam dunkel).

Beispiel 5

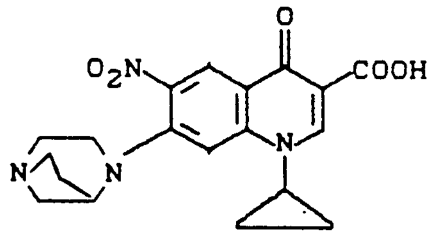

x HCl

Man setzt analog Beispiel 1 7-Chlor-1-cyclopropyl-1,4-dihydro-6-nitro-4-oxo-3-chinolincarbonsäure um und erhält 1-Cyclopropyl-7-(1,4-diazabicyclo[3.2.1]oct-4-yl)-1,4-dihydro-6-nitro-4-oxo-3-chinolincarbonsäure-Hydrochlorid vom Schmelzpunkt 303-307°C (unter Zersetzung).

Beispiel 6

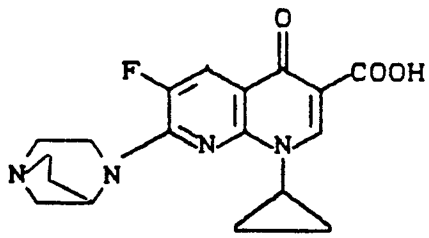

x HCl

Man setzt analog Beispiel 1 7-Chlor-1-cyclopropyl-6-fluor-1,4-dihydro-4-oxo-1,8-naphthyridin-3-carbonsäure um und erhält 1-Cyclopropyl-7-(1,4-diazabicyclo[3.2.1]oct-4-yl)-6-fluor-1,4-dihydro-4-oxo-1,8-naphthyridin-3-carbonsäure-Hydrochlorid vom Schmelzpunkt >300°C unter Zersetzung.

Beispiel 7

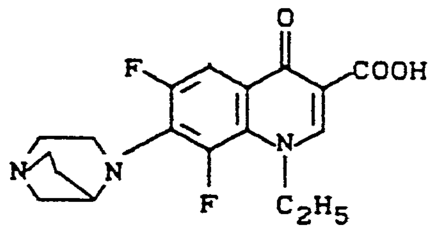

x HCl

Man setzt analog Beispiel 1 1-Ethyl-6,7,8-trifluor-1,4-dihydro-4-oxo-3-chinolincarbonsäure um und erhält 7-(1,4-Diazabicyclo[3.2.1]oct-4-yl)-1-ethyl-6,8-difluor-1,4-dihydro-4-oxo-3-chinolincarbonsäure-Hydrochlorid vom Schmelzpunkt 308-312°C unter Zersetzung.

Beispiel 8

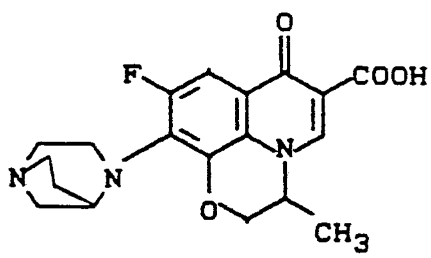

x HCl

Eine Mischung aus 2,8 g (10 mmol) 9,10-Difluor-2,3-dihydro-3-methyl-7-oxo-7H-pyrido[1,2,3-de][1,4]benzoxacin-6-carbonsäure und 1,2 g (10,7 mmol) 1,4-Diazabicyclo[3.2.1]-octan in 25 ml Dimethylsulfoxid wird mit 2,2 g (20 mmol) 1,4-Diazabicyclo[2.2.2]octan versetzt und 5 Stunden auf 120°C erhitzt. Die Mischung wird im Vakuum eingeengt, der

Rückstand mit 40 ml Acetonitril verrührt, der ungelöste Rückstand abgesaugt und durch Chromatographie an Kieselgel mit Dichlormethan/Methanol/20 % wäßrige Ammoniaklösung (2:4:1) als Laufmittel gereinigt. Man isoliert 1,2 g Festprodukt, das durch Lösen in 8 ml halbkonzentrierter Salzsäure und Ausfällen mit 30 ml Ethanol ins Hydrochlorid überführt wird.
Ausbeute: 1,1 g 10-(1,4-Diazabicyclo[3.2.1]oct-4-yl)-9-fluor-2,3-dihydro-3-methyl-7-oxo-7H-pyrido[1,2,3-de][1,4]-benzoxacin-6-carbonsäure-Hydrochlorid vom Schmelzpunkt 355°C unter Zersetzung (wird ab etwa 290°C dunkel).

Beispiel 9

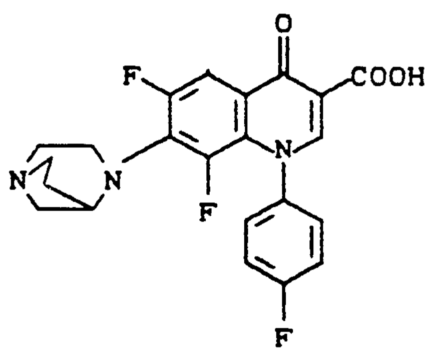

x HCl

Man setzt analog Beispiel 1 6,7,8-Trifluor-1-(4-fluorphenyl)-1,4-dihydro-4-oxo-3-chinolincarbonsäure um und erhält 7-(1,4-Diazabicyclo[3.2.1]oct-4-yl)-6,8-difluor-1-(4-fluorphenyl)-1,4-dihydro-4-oxo-3-chinolincarbonsäure-Hydrochlorid vom Schmelzpunkt 310-314°C unter Zersetzung.

Beispiel 10

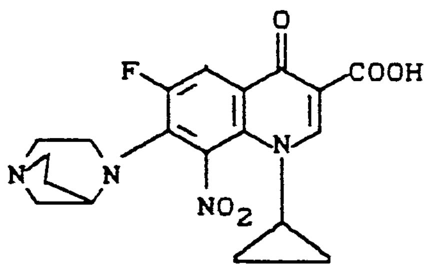

2,0 g (6 mmol) 7-Chlor-1-cyclopropyl-6-fluor-1,4-dihydro-8-nitro-4-oxo-3-chinolincarbonsäure werden in einer Mischung aus 10 ml Acetonitril und 25 ml Dimethylformamid mit 0,7 g (6 mmol) 1,4-Diazabicyclo[2.2.2]octan und 0,7 g (6 mmol) 1,4-Diazabicyclo[3.2.1]octan versetzt und 2 Stunden unter Rückfluß erhitzt. Anschließend wird eingeengt und mit Wasser versetzt. Man läßt unter Eiskühlung auskristallisieren, saugt ab, wäscht mit Wasser und trocknet.

Ausbeute: 1 g (41 % der Theorie) 1-Cyclopropyl-7-(1,4-diazabicyclo[3.2.1]oct-4-yl)-6-fluor-1,4-dihydro-8-nitro-4-oxo-3-chinolincarbonsäure vom Schmelzpunkt 215-232° C (unter Zersetzung). Nach Umkristallisation aus Glykolmonomethylether/Ethanol beträgt der Schmelzpunkt 224-232° C (unter Zersetzung).

Die hierfür benötigte 7-Chlor-1-cyclopropyl-6-fluor-1,4-dihydro-8-nitro-4-oxo-3-chinolincarbonsäure wird über folgende Stufen erhalten:

a) 2,4-Dichlor-5-fluor-3-nitro-benzoesäure

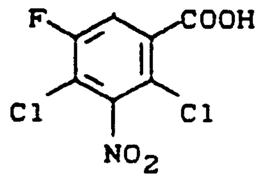

Unter Eiskühlung und Rühren werden 34 ml konzentrierter Schwefelsäure tropfenweise mit 40 ml konzentrierter Salpetersäure versetzt. In dieses Nitriergemisch trägt man portionsweise 20,9 g 2,4-Dichlor-5-fluorbenzoesäure ein, wobei die Temperatur auf 45 - 50°C steigt. Dann wird noch 3 Stunden auf 90 - 100°C erhitzt, das auf Raumtemperatur abgekühlte Gemisch auf 350 ml Eiswasser gegossen, der Niederschlag abgesaugt und mit Wasser gewaschen. Das feuchte Rohprodukt wird in 30 ml Methanol heiß gelöst und die Lösung mit 150 ml Wasser versetzt.

Der Niederschlag wird kalt abgesaugt, mit Methanol/Wasser gewaschen und im Vakuum bei 80°C getrocknet. Es werden 21,2 g rohe 2,4-Dichlor-5-fluor-3-nitrobenzoesäure erhalten. Sie ist genügend rein für die weiteren Umsetzungen. Eine Probe aus Toluol/Petrolether umkristallisiert liefert Kristalle vom Schmelzpunkt 192°C.

b) 2,4-Dichlor-5-fluor-3-nitro-benzoylchlorid

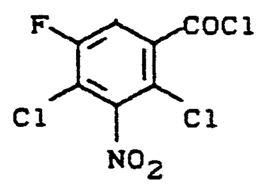

106,6 g 2,4-Dichlor-5-fluor-3-nitro-benzoesäure werden mit 250 ml Thionylchlorid 2 Stunden unter Rückfluß zum Sieden erhitzt. Das überschüssige Thionylchlorid wird dann bei Normaldruck abdestilliert und der Rückstand im Feinvakuum fraktioniert. Bei 110 - 115°C/0,08-0,09 mbar gehen 104,7 g 2,4-Dichlor-5-fluor-3-nitro-benzoylchlorid über. Beim Stehen bilden sich Kristalle vom Schmelzpunkt 35 - 37°C.

c) (2,4-Dichlor-5-fluor-3-nitro-benzoyl)-essigsäure-ethylester

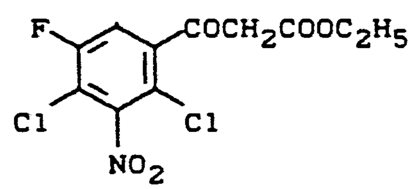

10,1 g Magnesiumspäne werden in 21 ml Ethanol mit 2,1 g Tetrachlormethan versetzt und nach Beginn der Wasserstoff-Entwicklung ein Gemisch aus 66,6 g Malonsäurediethylester, 40 ml Ethanol und 150 ml Toluol bei 50 - 60°C tropfenweise zugefügt. Man rührt 1 Stunde bei dieser Temperatur nach, kühlt auf -5 bis -10°C und tropft langsam eine Lösung von 109,2 g 2,4-Dichlor-5-fluor-3-nitro-benzoylchlorid in 50 ml Toluol zu. Danach wird 1 Stunde bei 0°C gerührt, über Nacht auf Raumtemperatur gebracht und noch 2 Stunden auf 40 - 50°C erwärmt. Das Reaktionsgemisch wird unter Eiskühlung mit einem Gemisch aus 160 ml Wasser und 10,4 ml konzentrierter Schwefelsäure versetzt und die organische Phase abgetrennt. Die wäßrige Phase wird mit Toluol extrahiert und der vereinigte organische Extrakt mit gesättigter Natriumchlorid-Lösung gewaschen, mit Natriumsulfat getrocknet und das Lösungsmittel abgezogen. Man erhält 144,5 g (2,4-Dichlor-5-fluor-3-nitro-benzoyl)-malonsäurediethylester als Rohprodukt. Dieses wird nach Zugabe von 200 ml Wasser und 0,6 g 4-Toluolsulfonsäure 3 Stunden unter Rückfluß erhitzt, die Mischung mit Methylenchlorid extrahiert, der Extrakt mit Natriumsulfat getrocknet und das Lösungsmittel im Vakuum abdestilliert. Es werden 118 g substituierter Benzoylessigester als Rohprodukt erhalten. Er besitzt eine für die weiteren Umsetzungen genügende Reinheit.

d) 7-Chlor-1-cyclopropyl-6-fluor-1,4-dihydro-8-nitro-4-oxo-3-chinolincarbonsäure

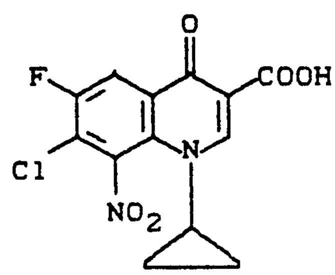

244,8 g (2,4-Dichlor-5-fluor-3-nitro-benzoyl)-essigsäureethylester werden mit 166 g Orthoameisensäuretriethylester und 185 g Essigsäureanhydrid 3 Stunden auf 150 -160°C erhitzt. Man engt im Vakuum ein und erhält 270 g 2-(2,4-Dichlor-5-fluor-3-nitro-benzoyl)-3-ethoxy-acrylsäure-ethylester als öligen Rückstand.

38 g dieser Zwischenstufe werden in 80 ml Ethanol unter Eiskühlung tropfenweise mit 5,9 g Cyclopropylamin versetzt und 1 Stunde bei 20°C gerührt. Das ausgefallene Produkt wird nach Zugabe von 100 ml Wasser abgesaugt, mit Ethanol/$H_2O$ (1:1) gewaschen und getrocknet. Man erhält 32,8 g 2-(2,4-Dichlor-5-fluor-3-nitro-benzoyl)-3-cyclopropylamino-acrylsäureethylester vom Schmelzpunkt 143 - 146°C.

7,8 g der vorgenannten Verbindung werden in 30 ml wasserfreiem Dioxan mit 3,1 g (1,8-Diazabicyclo[5.4.0]undec-7-en (DBU) versetzt und 4 Stunden auf 100°C erhitzt. Das Lösungsmittel wird im Vakuum abdestilliert, der Rückstand in Methylenchlorid/Wasser aufgenommen, die Methylenchlorid-Phase abgetrennt, mit Natriumsulfat getrocknet und das Methylenchlorid abdestilliert. Es werden 7,2 g 7-Chlor-1-cyclopropyl-6-fluor-1,4-dihydro-8-nitro-4-oxo-3-chinolincarbonsäureethylester als Rohprodukt erhalten. Nach Umkristallisation aus Acetonitril schmelzen die hellbraunen Kristalle bei 174 - 175°C.
Ausbeute: 6 g.

35,4 g dieses Esters werden in einem Gemisch aus 195 ml Essigsäure, 142 ml Wasser und 22 ml konzentrierter Schwefelsäure 1,5 Stunden auf 150°C erhitzt. Die Lösung wird in 500 ml Eiswasser gegossen, der Niederschlag abgesaugt, mit Wasser gewaschen und im Vakuum getrocknet. Ausbeute: 31,8 g 7-Chlor-1-cyclopropyl-6-fluor-1,4-dihydro-8-nitro-4-oxo-3-chinolincarbonsäure vom Schmelzpunkt 260 - 261°C.

Beispiel 11

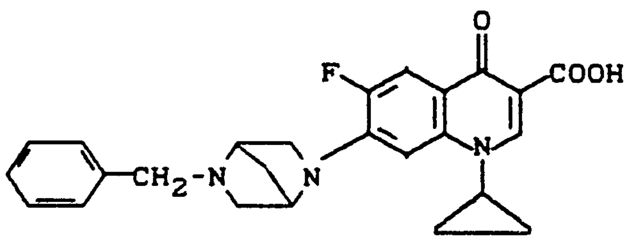

530 mg (2 mmol) 1-Cyclopropyl-6,7-difluor-1,4-dihydro-4-oxo-3-chinolincarbonsäure werden in 4 ml Acetonitril und 6 ml Dimethylformamid mit 530 mg (2 mmol) 2-Benzyl-2,5-diazabicyclo[2.2.1]heptan-Dihydrochlorid und 880 mg (7,9 mmol) 1,4-Diazabicyclo[2.2.2]octan 6 Stunden unter Rückfluß erhitzt. Das Reaktionsgemisch wird eingeengt, der Rückstand mit Wasser verrührt, der Niederschlag abgesaugt, mit Wasser gewaschen und getrocknet. Das erhaltene Rohprodukt (0,8 g vom Schmelzpunkt 194 - 205°C und Zersetzung) wird mit Dichlormethan/Methanol/17 % wäßriger Ammoniumhydroxidlösung (150:40:1) als Laufmittel an 60 g Kieselgel chromatographisch gereinigt. Man erhält 0,57 g (66 % der Theorie) 7-(5-Benzyl-2,5-diazabicyclo[2.2.1]hept-2-yl)-1-cyclopropyl-6-fluor-1,4-dihydro-4-oxo-3-chinolincarbonsäure vom Schmelzpunkt 205 - 214°C (unter Zersetzung).

Beispiel 12

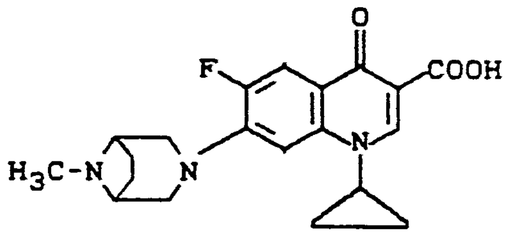

Analog Beispiel 11 wird mit 8-Methyl-3,8-diazabicyclo-[3.2.1]octan-Dihydrochlorid umgesetzt und das Rohprodukt aus Glykolmonomethylether umkristallisiert. Man erhält 1-Cyclopropyl-6-fluor-1,4-dihydro-7-(8-methyl-3,8-diazabicyclo[3.2.1]oct-3-yl)-4-oxo-3-chinolincarbonsäure vom Schmelzpunkt 273 - 278° C (unter Zersetzung).

Beispiel 13

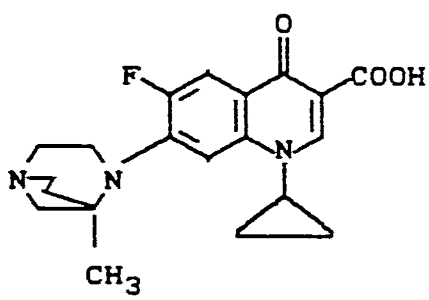

• HCl

Man setzt analog Beispiel 1 mit 5-Methyl-1,4-diazabicyclo-[3.2.1]octan um und erhält 1-Cyclopropyl-6-fluor-1,4-dihydro-7-(5-methyl-1,4-diazabicyclo[3.2.1]oct-4-yl)-4-oxo-3-chinolincarbonsäure-Hydrochlorid vom Schmelzpunkt >300° C (unter Zersetzung).

Das hierfür verwendete 5-Methyl-1,4-diazabicyclo[3.2.1]-octan wird über folgende Stufen erhalten:

a) 3-Methyl-3-methoxycarbonylmethylpiperazin-2-on

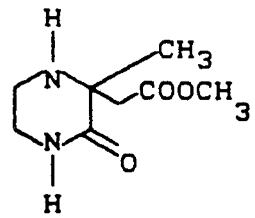

Man löst unter Rühren 160 g (1 mol) Citraconsäuredimethylester in 300 ml absolutem Ether und fügt 66 g (1,1 mol) Ethylendiamin hinzu. Die Lösung kommt nach kurzer Zeit zum Rückfluß und wird über Nacht bei Raumtemperatur gerührt. Das ausgefallene Produkt wird abgesaugt und aus Dioxan umkristallisiert.

Ausbeute: 125 g (67 % der Theorie)
Schmelzpunkt: 135 - 145°C (aus Dioxan)

b) 2-(2-Hydroxyethyl)-2-methylpiperazin

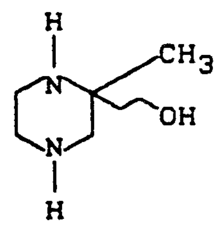

Zu einer Suspension von 30,4 g (0,8 mol) Lithiumaluminiumhydrid in 300 ml absolutem Tetrahydrofuran tropft man eine heiße Lösung von 74,6 g (0,4 mol)

3-Methyl-3-methoxycarbonylmethylpiperazin-2-on in 300 ml absolutem Dioxan. Anschließend rührt man 16 Stunden unter Rückfluß.

Dann wird abgekühlt, mit 30 ml Wasser, 30 ml 15 %iger wäßriger Kalilauge und wieder 30 ml Wasser das überschüssige $LiAlH_4$ zerstört und die anorganischen Salze abgesaugt. Diese werden noch fünfmal mit Chloroform gründlich durchgerührt. Die organischen Phasen werden über Kaliumcarbonat getrocknet, eingeengt und destilliert.

Ausbeute: 45 g (78 % der Theorie)
Siedepunkt: $78^0$ C/0,05 mbar.

c) 2-(2-Chlorethyl)-2-methylpiperazin-Dihydrochlorid

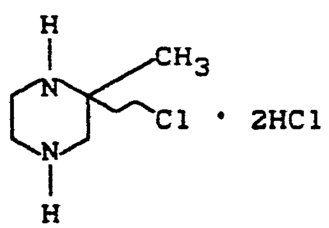

Zu 363 g (3 mol) Thionylchlorid in 500 ml Chloroform tropft man unter Erhitzen zum Rückfluß und Rühren 110 g (0,76 mol) 2-(2-Hydroxyethyl)-2-methylpiperazin in 600 ml Chloroform. Dann wird bis zum Ende der $SO_2$-Entwicklung unter Rückfluß erhitzt.

Man kühlt ab, tropft 500 ml Wasser hinzu, trennt die wäßrige Phase ab und wäscht die Chloroform-Phase zweimal mit 200 ml Wasser. Die wäßrigen Phasen werden zur Trockene eingedampft, in 1 l Wasser aufgenommen und mit Aktivkohle 30 Minuten zum Rückfluß erhitzt.

Man filtriert, engt zur Trockene ein, wäscht die Kristalle mit kaltem Methanol, saugt ab und trocknet im Vakuumexiccator über Phosphorpentoxid.

Ausbeute: 160 g (89,4 % der Theorie)
Schmelzpunkt: >300° C.

d) 5-Methyl-1,4-diazabicyclo[3.2.1]octan

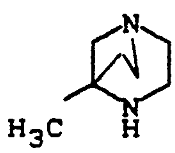

Man löst 100 g (0,42 Mol) 2-(2-Chlorethyl)-2-methylpiperazin · 2HCl in 100 ml Wasser und tropft unter Eiskühlung eine Lösung von 70 g (1,75 Mol) Natriumhydroxid in 70 ml Wasser hinzu. Die Temperatur wird unter 40° C gehalten. Anschließend erhitzt man 1 Stunde auf 80° C. Die Lösung wird mit Wasser versetzt, um ausgeschiedenes Natriumchlorid aufzulösen und mit Kaliumcarbonat gesättigt. Dann wird mehrfach mit Chloroform extrahiert, die organischen Phasen über Kaliumcarbonat getrocknet, eingeengt und destilliert.

Ausbeute: 45 g (85 % der Theorie)
Siedepunkt: 60° C/6 mbar.

Beispiel 14

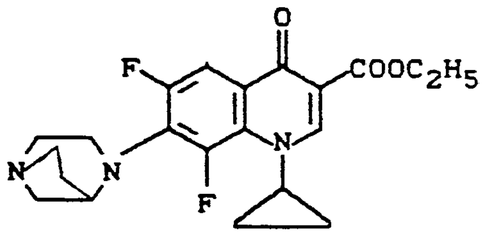

3,75 g (10 mmol) 1-Cyclopropyl-7-(1,4-diazabicyclo[3.2.1]-oct-4-yl)-6,8-difluor-1,4-dihydro-4-oxo-3-chinolincarbonsäure werden in 80 ml Ethanol suspendiert und bei Rückflußtemperatur ein trockener Chlorwasserstoffstrom eingeleitet. Nach beendeter Umsetzung wird eingeengt, der Rückstand in Wasser gelöst, mit Natriumcarbonat-Lösung auf pH 8 eingestellt, mit Dichlormethan extrahiert, mit Natriumsulfat getrocknet und das Filtrat eingeengt. Der Rückstand wird aus Ethanol/Wasser umkristallisiert. Man erhält 1,1 g 1-Cyclopropyl-7-(1,4-diazabicyclo[3.2.1]-oct-4-yl)-6,8-difluor-1,4-dihydro-4-oxo-3-chinolincarbonsäureethylester vom Schmelzpunkt 196-199° C.

0230274

Beispiel 15

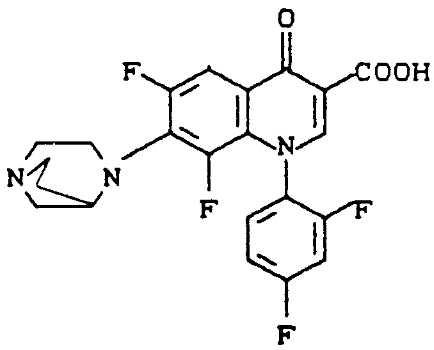

x HCl

Man setzt analog Beispiel 1 6,7,8-Trifluor-1-(2,4-difluorphenyl)-1,4-dihydro-4-oxo-3-chinolincarbonsäure um und erhält 7-(1,4-Diazabicyclo [3.2.1]oct-4-yl)-6,8-difluor-1-(2,4-difluorphenyl)-1,4-dihydro-4-oxo-3-chinolincarbonsäure-Hydrochlorid vom Schmelzpunkt 329-331° C unter Zersetzung.

Beispiel 16

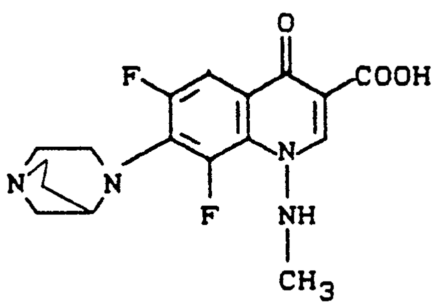

x HCl

Man setzt analog Beispiel 1 6,7,8-Trifluor-1,4-dihydro-1-methylamino-4-oxo-3-chinolincarbonsäure um und erhält 7-(1,4-Diazabicyclo[3.2.1]oct-4-yl)-6,8-difluor-1,4-dihydro-1-methylamino-4-oxo-3-chinolincarbonsäure-Hydrochlorid vom Schmelzpunkt 300-305° C unter Zersetzung.

Beispiel 17

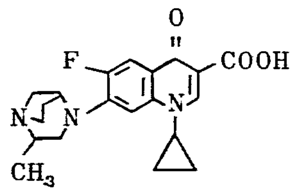

2,65 g (10 mmol) 1-Cyclopropyl-6,7-difluor-1,4-dihydro-4-oxo-3-chinolincarbonsäure werden in 20 ml Acetonitril und 10 ml Dimethylformamid mit 2,7 g (24 mmol) 1,4-Diazabicyclo[2.2.2]octan und 2,1 g (17 mmol) 2-Methyl-1,4-diazabicyclo[3,2,1]octan versetzt und 6 Stunden unter Rückfluß erhitzt. Die Mischung wird eingedampft, der Rückstand mit Wasser versetzt (pH 7), das ungelöste Kristallisat abgesaugt, mit Wasser gewaschen und aus Glykolmonomethylether umkristallisiert. Man erhält 1,4 g 1-Cyclopropyl-6-fluor-1,4-dihydro-7-(2-methyl-1,4-diazabicyclo[3.2.1]-oct-4-yl)-4-oxo-3-chinolincarbonsäure vom Schmelzpunkt 255-257°C (unter Zersetzung).

Das hierfür benötigte 2-Methyl-1,4-diazabicyclo[3.2.1]octan wird auf folgendem Wege erhalten:

5-Methyl-2-methoxycarbonylmethylpiperazin-3-on

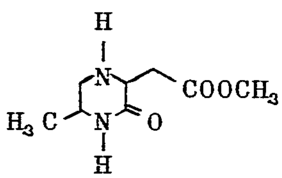

Man mischt 81 g (1,1 mol) 1,2-Propylendiamin, 144 g (1 mol) Maleinsäuredimethylester und 250 ml absoluten Ether. Die Mischung kommt zum Rückfluß und wird gelegentlich mit Eiswasser gekühlt. Anschließend rührt man über Nacht bei Raumtemperatur. Man saugt ab, wäscht mit wenig eiskaltem Isopropanol und trocknet an der

Luft. Die Mutterlaugen werden eingeengt und der Rückstand aus Essigester umkristallisiert.
Ausbeute: 95 g (51 % d. Th.)
Schmp. : 102 - 105 °C

2-(2-Hydroxyethyl)-5-methylpiperazin

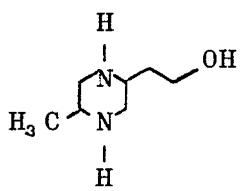

Zu einer Suspension von 30,4 g (0,8 mol) $LiAlH_4$ in 300 ml abs. Tetrahydrofuran tropft man eine Lösung von 74,5 g (0,4 mol) 5-Methyl-2-methoxycarbonylmethylpiperazin-3-on in 300 ml abs. Tetrahydrofuran. Anschließend erhitzt man 16 Stdn. unter Rückfluß. Dann zersetzt man überschüssiges $LiAlH_4$ nacheinander mit 30 ml Wasser, 30 ml 15 %iger Kalilauge und 30 ml Wasser, saugt die anorganischen Salze ab und rührt sie mehrfach mit Dichlormethan durch. Die organischen Phasen werden über $K_2CO_3$ getrocknet, eingeengt und destilliert.
Ausbeute: 31,8 g (55,1 % d. Th.)
Siedepunkt: 85 - 87 °C/0,07 mbar

2-(2-Chlorethyl)-5-methylpiperazin-Dihydrochlorid

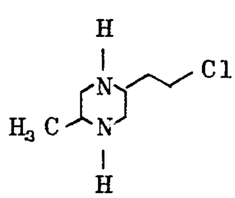
· 2HCl

Zu 95 g (0,79 mol) Thionylchlorid in 125 ml Methylenchlorid tropft man unter Erhitzen zum Rückfluß 28 g (0,208 mol) 2-(2-Hydroxyethyl)-5-methylpiperazin in 125 ml Dichlormethan. Anschließend rührt man 5 Stdn. unter Rückfluß. Dann tropft man 200 ml Wasser vorsichtig hinzu und trennt die wäßrige Phase ab. Die organische Phase wird zweimal mit Wasser gewaschen und verworfen. Die wäßrigen Phasen werden eingeengt, erneut in 200 ml Wasser aufgenommen und mit 3 g Aktiv-Kohle 30 Min. zum Rückfluß erhitzt. Man filtriert, engt zur Trockne ein, versetzt mit Methanol, saugt ab und trocknet im Vakuumexsikkator $P_4O_{10}$.
Ausbeute: 23,5 g (48 % d. Th.)

2-Methyl-1,4-diazabicyclo[3,2,1]octan

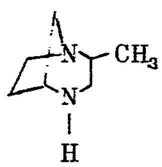

Man löst 23,5 g (0,1 mol) 2-(2-Chlorethyl)-5-methylpiperazin-Dihydrochlorid in 20 ml Wasser und tropft bei maximal 40°C 32 g (0,4 mol) 50%-ige Natronlauge hinzu. Dann rührt man 1 Std. bei 75°C, kühlt, sättigt mit Kaliumcarbonat und extrahiert zehnmal mit je 50 ml Dichlormethan. Die organischen Phasen werden über Kaliumcarbonat getrocknet, eingeengt und destilliert.
Ausbeute: 9,7 g (76,8 % d. Th.)
Siedepunkt: 60 - 65 °C/0,8 mbar

Beispiel 18

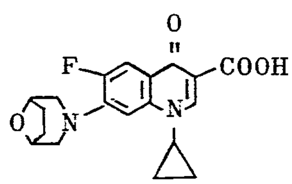

Analog Beispiel 17 wird 8-Oxa-3-aza-bicyclo[3,2,1]octan-hydrochlorid umgesetzt, wobei 1-Cyclopropyl-6-fluor-1,4-dihydro-7-(8-oxa-3-aza-bicyclo [3,2,1] oct-3-yl)-4-oxo-3-chinolincarbonsäure vom Schmelzpunkt 272 - 275 °C (unter Zersetzung) (aus Glykolmonomethylether) erhalten wird.

Das hierfür benötigte 8-Oxa-3-aza-bicyclo [3,2,1] octan-hydrochlorid wird auf folgendem Weg erhalten:

3-Benzyl-8-oxa-3-azabicyclo [3,2,1] octan

61,2 g (0,15 mol) 2,5-Bis-(tosyloxymethyl)-tetrahydrofuran (F. H. Newth, L. F. Wiggins, J. Chem. Soc. 155 (1948)) 300 ml Toluol und 54 g (0,5 mol) Benzylamin werden 20 Stdn. unter Rückfluß erhitzt. Man filtriert, wäscht mit Toluol, engt ein und destilliert.
Ausbeute : 21,5 g (70,5 % d. Th.)
Siedepunkt: 140 °C/9mbar

8-Oxa-3-azabicyclo[3,2,1] octan-Hydrochlorid

Man löst 20 g (0,098 mol) 3-Benzyl-8-oxa-3-azabicyclo [3,2,1] octan in 150 ml Methanol und setzt 8,5 ml konzentrierte Salzsäure hinzu. Man hydriert an 5 g Pd-Kohle (10 %ig) bei 50 °C und 50 bar. Der Katalysator wird abgesaugt, die Lösung eingeengt, der Rückstand mit Aceton verrieben, abfiltriert und über $P_4O_{10}$ getrocknet.
Ausbeute : 12,5 g (85,3 % d. Th.)
Schmp. : 202 - 204 °C

Beispiel 19

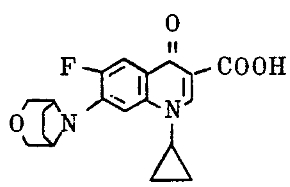

Analog Beispiel 17 wird 3-Oxa-8-aza-bicyclo [3,2,1] octan-hydrochlorid umgesetzt, wobei 1-Cyclopropyl-6-fluor-1,4-dihydro-7-(3-oxo-8-aza-bicyclo [3,2,1] oct-8-yl)-4-oxo-3-chinolincarbonsäure vom Schmelzpunkt 283 - 284 °C (unter Zersetzung) (aus Glykolmonomethylether) erhalten wird.

Das hierfür benötigte 3-Oxa-8-aza-bicyclo [3,2,1] octan wird auf folgendem Weg erhalten:

3-Oxa-8-azabicyclo [3,2,1] octan-Hydrochlorid

Man löst 22,7 g (0,104 mol) 8-Benzyl-3-oxa-8-azabicyclo [3,2,1]-octan (J. von Braun, J. Seemann, Ber. Deutsch. Chem. Ges. 56, 1840 (1923)) in 140 ml Methanol und setzt 9,3 ml konzentrierte Salzsäure hinzu. Dann hydriert man an 5 g Pd-Kohle (5 %) bei 60 °C und 60 bar. Der Katalysator wird abgesaugt, die Lösung eingeengt, der Rückstand mit Aceton verrieben, abgesaugt und im Exsikkator über $P_4O_{10}$ getrocknet.
Ausbeute: 13,7 g (88,7 % d. Th.)
Schmp. : 236 °C

Beispiel 20

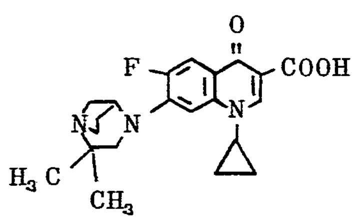

Analog Beispiel 17 wird 2,2-Dimethyl-1,4-diazabicyclo [3.2.1] octan umgesetzt, wobei 1-Cyclopropyl-6-fluor-1,4-dihydro-7-(2,2-dimethyl-1,4-diazabicyclo [3.2.1] oct-4-yl)-4-oxo-3-chinolincarbonsäure vom Schmelzpunkt 242-246 °C (unter Zersetzung) erhalten wird.

Das hierfür benötigte 2,2-Dimethyl-1,4-diazabicyclo [3.2.1] octan wird auf folgendem Wege erhalten:

5,5-Dimethyl-2-methoxycarbonylmethylpiperazin-3-on

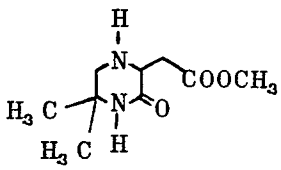

Man mischt 80 g (0,91 mol) 1,2-Diamino-2-methylpropan, 127 g (0,88 ml) Maleinsäuredimethylester und 200 ml absoluten Ether. Nach der exothermen Reaktion rührt man noch 16 Stunden, saugt ab, engt die Mutterlauge ein und kristallisiert aus Diisopropylether um.

Ausbeute : 154 g (87 % der Theorie)
Schmelzpunkt: 76-78 $^{o}C$

5,5-Dimethyl-2-(2-hydroxyethyl)-piperazin

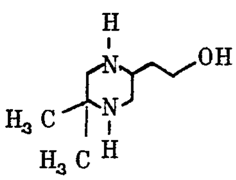

Zu einer Suspension von 30,4 g (0,4 mol) $LiAlH_4$ in 300 ml Tetrahydrofuran tropft man eine heiße Lösung von 80 g (0,4 mol) 5,5-Dimethyl-2-methoxycarbonylmethylpiperazin-3-on in 300 ml Tetrahydrofuran. Anschließend kocht man 16 Stunden unter Rückfluß. Nach Abkühlen zersetzt man überschüssiges $LiAlH_4$ nacheinander mit 30 ml Wasser, 30 ml 15 %iger Kalilauge und 30 ml Wasser. Man saugt die anorganischen Salze ab, rührt sie mehrfach mit Dichlormethan aus und trocknet die organischen Phasen über Kaliumcarbonat. Dann wird eingeengt und destilliert.

Ausbeute : 31,6 g (49,9 % der Theorie)
Siedepunkt : 114 $^{o}C$/0,1 mbar

2-(2-Chlorethyl)-5,5-dimethylpiperazin-Dihydrochlorid

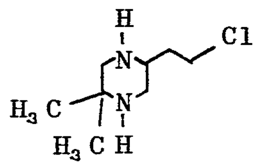

20 g (0,126 mol) 5,5-Dimethyl-2-(2-hydroxyethyl)-piperazin in 100 ml Dichlormethan tropft man zu unter Rückfluß erhitzten 60 g (0,5 mol) Thionylchlorid in 100 ml Dichlormethan. Anschließend rührt man noch 2 Stunden unter Rückfluß. Man hydrolysiert mit 100 ml Wasser und trennt die wäßrige Phase ab. Die organische Phase wird zweimal mit Wasser gewaschen und verworfen. Die wäßrigen Phasen werden zur Trockene eingeengt, in 200 ml Wasser aufgenommen und mit 3 g Aktivkohle 30 Minuten unter Rückfluß erhitzt. Man filtriert, engt ein, wäscht den kristallinen Rückstand mit wenig kaltem Methanol und trocknet im Exsikkator über $P_4O_{10}$.

Ausbeute: 21 g (66,8 % der Theorie)

2,2-Dimethyl-1,4-diazabicyclo[3.2.1]octan

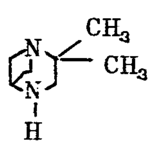

Zu 20 g (0,08 mol) 2-(2-Chlorethyl)-5,5-dimethylpiperazin-Dihydrochlorid in 20 ml Wasser tropft man bei maximal 40 °C 30 g 50 %ige Natronlauge. Anschließend erhitzt man noch 1 Stunde auf 80 °C. Dann kühlt man ab, sättigt mit Kaliumcarbonat, extrahiert zehnmal mit je 50 ml Dichlormethan, trocknet über Kaliumcarbonat, engt ein und destilliert.

Ausbeute : 7,9 g (70,5 % der Theorie)
Siedepunkt: 65 °C/4 mbar

Beispiel 21

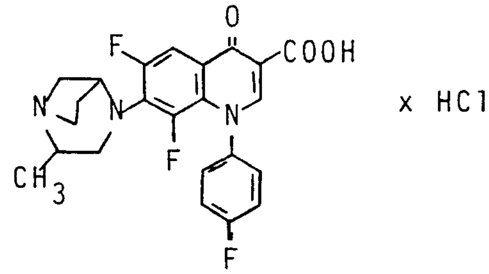

3,37 g (10 mmol) 6,7,8-Trifluor-1-(4-fluor-phenyl)-1,4-dihydro-4-oxo-3-chinoloncarbonsäure werden in 30 ml Dimethylsulfoxid mit 2,7 g (24 mmol) 1,4-Diazabicyclo/2.2.2/octan und 2,1 g (17 mmol) 2-Methyl-1,4-diazabicyclo/3.2.1/octan versetzt und 2 Stunden auf 140°C erhitzt. Das Lösungsmittel wird im Hochvakuum abdestilliert und der Rückstand mit Wasser versetzt (pH 7). Dann wird mit halbkonzentrierter Salzsäure auf pH 1 gestellt, mit dem gleichen Volumen Ethanol versetzt und das Hydrochlorid kalt abgesaugt. Nach Umkristallisation aus Wasser/Ethanol werden 2,5 g 6,8-Difluor-1-(4-fluor-phenyl)-1,4-dihydro-7-(2-methyl-1,4-diazabicyclo-/3.2.1/-oct-4-yl)-4-oxo-3-chinolincarbonsäure-hydrochlorid vom Schmelzpunkt 325-330°C (Zers.) erhalten.

Beispiel 22

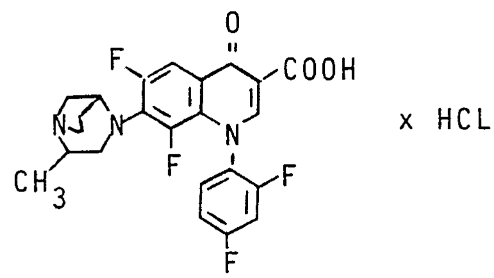

3,5 g (10 mmol) 6,7,8-Trifluor-1-(2,4-difluor-phenyl)-1,4-dihydro-4-oxo-3-chinoloncarbonsäure werden in 30 ml Dimethylsulfoxid mit 2,7 g (24 mmol) 1,4-Diazabicyclo/2.2.2/octan und 2,1 g (17 mmol) 2-Methyl-1,4-diazabicyclo/3.2.1/octan

2 Stunden auf 140°C erhitzt. Die Aufarbeitung erfolgt wie in Beispiel 21. Nach Umkristallisation aus Wasser werden 1,1 g 6,8-Difluor-1-(2,4-difluor-phenyl)-1,4-dihydro-7-(2-methyl-1,4-diazabicyclo/3.2.1/oct-4-yl)-4-oxo-3-chinolincarbonsäurehydrochlorid vom Schmelzpunkt 278-280°C (Zers.) erhalten.

Beispiel 23

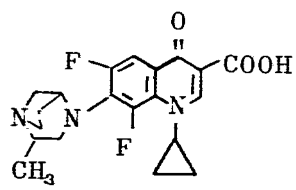

Analog Beispiel 17 werden 2,8 g (10 mmol) 1-Cyclopropyl-6,7,8-trifluor-1,4-dihydro-4-oxo-3-chinolincarbonsäure umgesetzt. Man erhält 1,3 g 1-Cyclopropyl-6,8-difluor-1,4-dihydro-7-(2-methyl-1,4-diazabicyclo[3.2.1]oct-4-yl)-4-oxo-3-chinolincarbonsäure vom Schmelzpunkt 239-242$^{0}$ (unter Zersetzung) (umkristallisiert aus Glykolmonomethylether).
Massenspektrum: $^{m}/_{e}$ 389 (M+), 325, 276, 247, 111, 97 (98 %), 69, 56 (98 %), 32, 28 (100 %).

## Patentansprüche

1. 7-(Azabicycloalkyl)-chinoloncarbonsäure- beziehungsweise -naphthyridoncarbonsäure-Derivate der Formel (I)

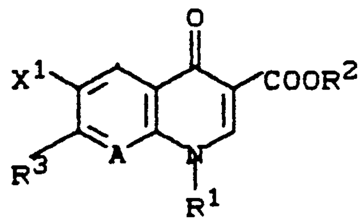

(I),

in welcher

$R^1$ für Methyl, Ethyl, Propyl, Isopropyl, Cyclopropyl, Vinyl, 2-Hydroxyethyl, 2-Fluorethyl, Methoxy, Amino, Methylamino, Dimethylamino, Phenyl, Fluorphenyl, 2,4-Difluorphenyl,

$R^2$ für Wasserstoff, Alkyl mit 1 bis 4 Kohlenstoffatomen, (5-Methyl-2-oxo-1,3-dioxol-4-yl)-methyl,

$R^3$ für einen Rest der Formel

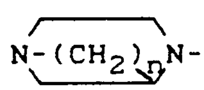 , 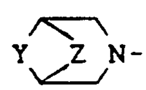 , 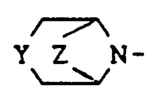 , 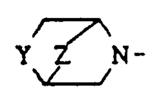 ,

in welcher

Y für $R^4$-N, O, S,

Z für $-(CH_2)_n-$, $-CH_2-O-CH_2-$, $-CH_2-S-CH_2-$, $-CH_2-S-$,

$R^5$
|
$-CH_2-N-CH_2-$,

n für 1, 2 oder 3,

$R^4$ für Wasserstoff, gegebenenfalls durch Hydroxy substituiertes Alkyl, Alkenyl oder Alkinyl mit 1 bis 4 Kohlenstoffatomen, gegebenenfalls durch Nitro oder Amino substituiertes Benzyl, Oxoalkyl mit 2 bis 4 Kohlenstoffatomen oder (5-Methyl-2-oxo-1,3-dioxol-4-yl)-methyl und

$R^5$ für Wasserstoff oder Methyl steht,

$X^1$ für Fluor, Chlor oder Nitro und

A für N oder C-$R^6$ steht, worin $R^6$ für Wasserstoff, Fluor, Chlor, Methyl oder Nitro steht oder auch gemeinsam mit $R^1$ eine Brücke der Struktur

$-O-CH_2-CH(|)-CH_3$, $-S-CH_2-CH(|)-CH_3$ oder

$-CH_2-CH_2-CH(|)-CH_3$

bilden kann,

mit der Maßgabe, daß für den Fall, daß $X^1$ für Fluor, $R^2$ für H und A für CF oder N stehen und $R^1$ für Ethyl, Cyclopropyl, Fluorethyl oder Vinyl steht oder $R^6$ mit $R^1$ eine Brücke der Struktur $-O-CH_2-CH-CH_3$ bildet, n ungleich 2 ist und $R^3$ nicht für den Rest

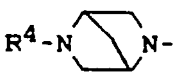 stehen kann, sowie ausgenommen die Verbindung 7-(2,5-Diazabicyclo[2.2.2]oct-2-yl)-1-ethyl-6-fluoro-1,4-dihydro-4-oxo-chinolincarbonsäure,

und deren pharmazeutisch verwendbaren Hydrate und Säureadditionssalze sowie die Alkali-, Erdalkali-, Silber- und Guanidiniumsalze der zugrundeliegenden Carbonsäuren.

2. 7-(Azabicycloalkyl)-chinoloncarbonsäure- beziehungsweise -napthyridoncarbonsäure-Derivate der Formel (I)

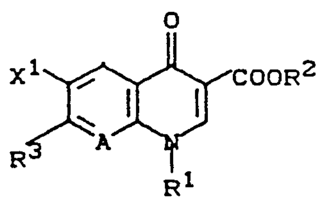

(I),

in welcher

$R^1$ für Methyl, Ethyl, Propyl, Isopropyl, Cyclopropyl, Vinyl, 2-Hydroxyethyl, 2-Fluorethyl, Methoxy, Amino, Methylamino, Dimethylamino, Phenyl, Fluorphenyl, 2,4-Difluorphenyl,

$R^2$ für Wasserstoff, Alkyl mit 1 bis 4 Kohlenstoffatomen, (5-Methyl-2-oxo-1,3-dioxol-4-yl)-methyl,

$R^3$ für einen im Ringsystem durch Hydroxy oder Methyl ein- oder mehrfach substituierten Rest der Formel

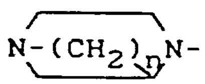 , 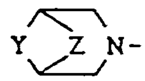 , 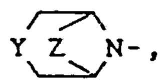 , 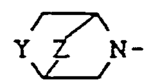 ,

in welcher

Y für $R^4$-N, O, S,

Z für $-(CH_2)_n-$, $-CH_2-O-CH_2-$, $-CH_2-S-CH_2-$, $-CH_2-S-$, $-CH_2-N(R^5)-CH_2-$,

n für 1, 2 oder 3,

$R^4$ für Wasserstoff, gegebenenfalls durch Hydroxy substituiertes Alkyl, Alkenyl oder Alkinyl mit 1 bis 4 Kohlenstoffatomen, gegebenenfalls durch Nitro oder Amino substituiertes Benzyl, Oxoalkyl mit 2 bis 4 Kohlenstoffatomen oder (5-Methyl-2-oxo-1,3-dioxol-4-yl)-methyl und

$R^5$ für Wasserstoff oder Methyl steht,

$X^1$ für Fluor, Chlor oder Nitro und

A für N oder C-$R^6$ steht, worin $R^6$ für Wasserstoff, Fluor, Chlor, Methyl oder Nitro steht oder auch gemeinsam mit $R^1$ eine Brücke der Struktur

$-O-CH_2-\underset{|}{C}H-CH_3$, $-S-CH_2-\underset{|}{C}H-CH_3$ oder

$-CH_2-CH_2-\underset{|}{C}H-CH_3$

bilden kann

und deren pharmazeutisch verwendbare Hydrate und Säureadditionssalze sowie die Alkali-, Erdalkali-, Silber- und Guanidiniumsalze der zugrundeliegenden Carbonsäuren.

3. 7-(Azabicycloalkyl)-chinoloncarbonsäure- bzw. -naphthyridoncarbonsäure-Derivate der Formel (I) gemäß Anspruch 1, in denen

$R^1$ für Methyl, Ethyl, Cyclopropyl, 2-Hydroxyethyl, Vinyl, 2-Fluorethyl, Methoxy, Methylamino, Phenyl, 4-Fluorphenyl, 2,4-Difluorphenyl,

$R^2$ für Wasserstoff, Methyl, Ethyl,

$R^3$ für einen Rest der Formel

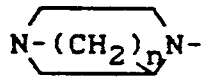 , 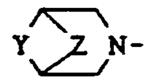 , 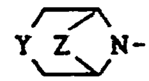 , 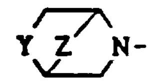 ,

in welcher

Y für $R^4$-N, O,

Z für $-(CH_2)_n-$, $-CH_2-O-CH_2-$, $-CH_2-N(R^5)-CH_2-$,

n für 1, 2 oder 3,

$R^4$ für Wasserstoff, gegebenenfalls durch Hydroxy substituiertes Alkyl, Alkenyl oder Alkinyl mit 1 bis 3 Kohlenstoffatomen, gegebenenfalls durch Nitro oder Amino substituiertes Benzyl oder Oxoalkyl mit 2 bis 4 Kohlenstoffatomen und

$R^5$ für Wasserstoff oder Methyl steht,

$X^1$ für Fluor, Chlor oder Nitro und

A für N oder C-$R^6$ steht, worin
$R^6$ für Wasserstoff, Fluor, Chlor, Methyl oder Nitro steht oder auch gemeinsam mit $R^1$ eine Brücke der Struktur

$-O-CH_2-CH(-)-CH_3$ oder $-CH_2-CH_2-CH(-)-CH_3$

bilden kann,

mit der Maßgabe, daß für den Fall, daß $X^1$ für Fluor, $R^2$ für H und A für CF oder N stehen und $R^1$ für Ethyl, Cyclopropyl, Fluorethyl oder Vinyl steht oder $R^6$ mit $R^1$ eine Brücke der Struktur $-O-CH_2-\underset{|}{C}H-CH_3$ bildet, n ungleich 2 ist und $R^3$ nicht für den Rest

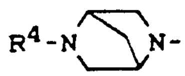 stehen kann, sowie ausgenommen die Verbindung 7-(2,5-Diazabicyclo[2.2.2]oct-2-yl)-1-ethyl-6-fluoro-1,4-dihydro-4-oxo-chinolincarbonsäure,

und deren pharmazeutisch verwendbaren Hydrate und Säureadditionssalze sowie die Alkali-, Erdalkali-, Silber- und Guanidiniumsalze der zugrundeliegenden Carbonsäuren.

4. 7-(Azabicycloalkyl)-chinoloncarbonsäure- bzw. -naphthyridoncarbonsäure-Derivate der Formel (I) gemäß Anspruch 1, in der

$R^1$ für Ethyl, Cyclopropyl, 2-Fluorethyl, Methylamino, Phenyl, 4-Fluorphenyl, 2,4-Difluorphenyl,

$R^2$ für Wasserstoff, Methyl, Ethyl,

$R^3$ für einen Rest der Formel

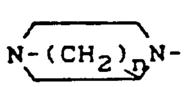 , 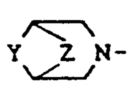 , 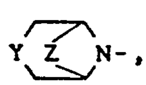 , 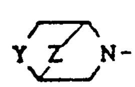 ,

in welcher

Y für $R^4$-N,

Z für $-(CH_2)_n-$,

n für 1 oder 2,

$R^4$ für Wasserstoff, gegebenenfalls durch Hydroxy substituiertes Alkyl, Alkenyl oder Alkinyl mit 1 bis 3 Kohlenstoffatomen, gegebenenfalls durch Nitro oder Amino substituiertes Benzyl oder Oxoalkyl mit 2 bis 4 Kohlenstoffatomen und

$R^5$ für Wasserstoff oder Methyl steht,

$X^1$ für Fluor, Chlor oder Nitro und

A für N oder C-$R^6$ steht, worin $R^6$ für Wasserstoff, Fluor, Chlor oder Nitro steht oder auch gemeinsam mit $R^1$ eine Brücke der Struktur

$-O-CH_2-\underset{|}{C}H-CH_3$ oder $-CH_2-CH_2-\underset{|}{C}H-CH_3$

bilden kann,

mit der Maßgabe, daß für den Fall, daß $X^1$ für Fluor, $R^2$ für H und A für CF oder N stehen und $R^1$ für Ethyl, Cyclopropyl, Fluorethyl oder Vinyl steht oder $R^6$ mit $R^1$ eine Brücke der Struktur $-O-CH_2-\underset{|}{C}H-CH_3$ bildet, n ungleich 2 ist und $R^3$ nicht für den Rest

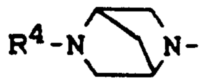 stehen kann, sowie ausgenommen die Verbindung 7-(2,5-Diazabicyclo[2.2.2]oct-2-yl)-1-ethyl-6-fluoro-1,4-dihydro-4-oxo-chinolincarbonsäure,

und deren pharmazeutisch verwendbaren Hydrate und Säureadditionssalze sowie die Alkali-, Erdalkali-, Silber- und Guanidiniumsalze der zugrundeliegenden Carbonsäuren.

5. Verfahren zur Herstellung von 7-(Azabicycloalkyl)-chinoloncarbonsäure- bzw. -naphthyridoncarbonsäure-Derivaten der allgemeinen Formel (I)

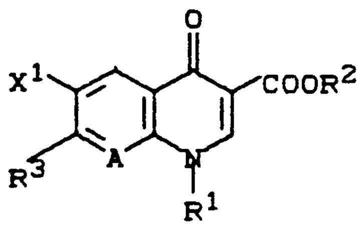

(I),

in welcher

$R^1$ für Methyl, Ethyl, Propyl, Isopropyl, Cyclopropyl, Vinyl, 2-Hydroxyethyl, 2-Fluorethyl, Methoxy, Amino, Methylamino, Dimethylamino, Phenyl, 4-Fluorphenyl, 2,4-Difluorphenyl,

$R^2$ für Wasserstoff, Alkyl mit 1 bis 4 Kohlenstoffatomen, (5-Methyl-2-oxo-1,3-dioxol-4-yl)-methyl,

$R^3$ für einen Rest der Formel

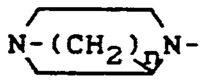 , 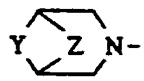 , 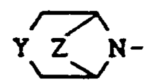 , 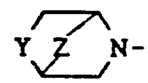 ,

in welcher

Y für $R^4$-N, O, S,

Z für $-(CH_2)_n-$, $-CH_2-O-CH_2-$, $-CH_2-S-CH_2-$, $-CH_2-S-$,

$$-CH_2-\overset{\overset{\displaystyle R^5}{|}}{N}-CH_2-,$$

n für 1, 2 oder 3,

$R^4$ für Wasserstoff, gegebenenfalls durch Hydroxy substituiertes Alkyl, Alkenyl oder Alkinyl mit 1 bis 4 Kohlenstoffatomen, gegebenenfalls durch Nitro oder Amino substituiertes Benzyl, Oxoalkyl mit 2 bis 4 Kohlenstoffatomen oder (5-Methyl-2-oxo-1,3-dioxol-4-yl)-methyl,

$R^5$ für Wasserstoff oder Methyl steht,

$X^1$ für Fluor, Chlor oder Nitro und

A für N oder C-$R^6$ steht, worin
$R^6$ für Wasserstoff, Fluor, Chlor, Methyl oder Nitro steht oder auch gemeinsam mit $R^1$ eine Brücke der Struktur
$-O-CH_2-CH(-)-CH_3$, $-S-CH_2-CH(-)-CH_3$ oder
$-CH_2-CH_2-CH(-)-CH_3$
bilden kann,

mit der Maßgabe, daß für den Fall, daß $X^1$ für Fluor, $R^2$ für H und A für CF oder N stehen und $R^1$ für Ethyl, Cyclopropyl, Fluorethyl oder Vinyl steht oder $R^6$ mit $R^1$ eine Brücke der Struktur $-O-CH_2-CH(-)-CH_3$ bildet, n ungleich 2 ist und $R^3$ nicht für den Rest

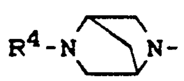 stehen kann, sowie ausgenommen die Verbindung 7-(2,5-Diazabicyclo[2.2.2]oct-2-yl)-1-ethyl-6-fluoro-1,4-dihydro-4-oxo-chinolincarbonsäure,

und deren pharmazeutisch verwendbaren Hydraten und Säureadditionssalzen sowie deren Alkali-, Erdalkali-, Silber- und Guanidiniumsalzen der zugrundeliegenden Carbonsäuren,

dadurch gekennzeichnet, daß man Verbindungen der Formel (II)

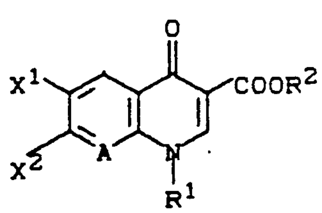

(II),

in welcher $R^1$, $R^2$, $X^1$ und A die oben angebenene Bedeutung haben und

$X^2$ für Halogen, insbesondere Fluor oder Chlor, steht,

mit Azabicycloalkanen der Formel (III)

$R^3$-H (III),

in welcher $R^3$ die oben angegebene Bedeutung hat, gegebenenfalls in Gegenwart von Säurebindungsmitteln umsetzt.

6. Verfahren zur Herstellung von 7-(Azabicycloalkyl)-chinoloncarbonsäure- bzw. -naphthyridoncarbonsäure-Derivaten der allgemeinen Formel (I)

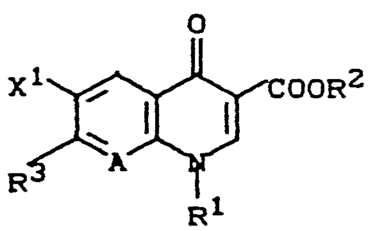

(I),

in welcher

$R^1$ für Methyl, Ethyl, Propyl, Isopropyl, Cyclopropyl, Vinyl, 2-Hydroxyethyl, 2-Fluorethyl, Methoxy, Amino, Methylamino, Dimethylamino, Phenyl, Fluorphenyl, 2,4-Difluorphenyl,

$R^2$ für Wasserstoff, Alkyl mit 1 bis 4 Kohlenstoffatomen, (5-Methyl-2-oxo-1,3-dioxol-4-yl)-methyl,

$R^3$ für einen im Ringsystem durch Hydroxy oder Methyl ein- oder mehrfach substituierten Rest der Formel

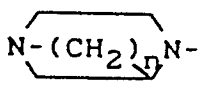 , 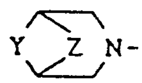 , 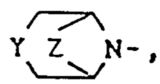 , 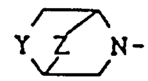 ,

in welcher

Y für $R^4$-N, O, S,

Z für $-(CH_2)_n-$, $-CH_2-O-CH_2-$, $-CH_2-S-CH_2-$, $-CH_2-S-$,

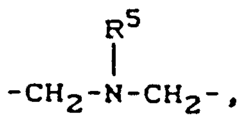

n für 1, 2 oder 3,

$R^4$ für Wasserstoff, gegebenenfalls durch Hydroxy substituiertes Alkyl, Alkenyl oder Alkinyl mit 1 bis 4 Kohlenstoffatomen, gegebenenfalls durch Nitro oder Amino substituiertes Benzyl, Oxoalkyl mit 2 bis 4 Kohlenstoffatomen oder (5-Methyl-2-oxo-1,3-dioxol-4-yl)-methyl und

$R^5$ für Wasserstoff oder Methyl steht,

$X^1$ für Fluor, Chlor oder Nitro und

A für N oder C-$R^6$ steht, worin
$R^6$ für Wasserstoff, Fluor, Chlor, Methyl oder Nitro steht oder auch gemeinsam mit $R^1$ eine Brücke der Struktur

$-O-CH_2-CH-CH_3$, $-S-CH_2-CH-CH_3$ oder

$-CH_2-CH_2-CH-CH_3$

bilden kann

und deren pharmazeutisch verwendbare Hydrate und Säureadditionssalze sowie die Alkali-, Erdalkali-, Silber- und Guanidiniumsalze der zugrundeliegenden Carbonsäuren,

**dadurch gekennzeichnet, daß man Verbindungen der Formel (II)**

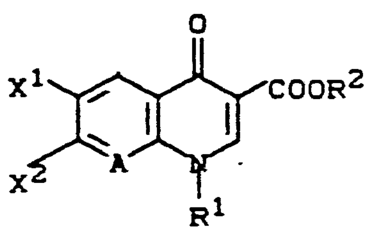

(II),

in welcher $R^1$, $R^2$, $X^1$ und A die oben angebenene Bedeutung haben und

$X^2$ für Halogen, insbesondere Fluor oder Chlor, steht,

mit Azabicycloalkanen der Formel (III)

$R^3$-H (III),

in welcher $R^3$ die oben angegebene Bedeutung hat, gegebenenfalls in Gegenwart von Säurebindungsmitteln umsetzt.

7. Verfahren zur Herstellung von 7-(Azabicycloalkyl)-chinoloncarbonsäure- beziehungsweise -naphthyridoncarbonsäure-Derivaten der allgemeinen Formel (I)

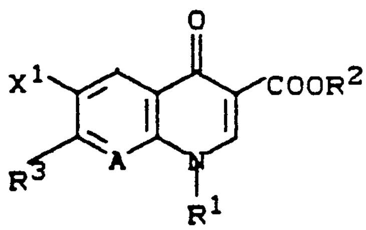

(I),

in welcher

$R^1$, $R^2$, A und $X^1$ die in Anspruch 4 angegebene Bedeutung haben und

$R^3$ für einen im Ringsystem gegebenenfalls durch Hydroxy oder Methyl substituierten Rest der Formel

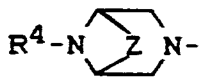 , 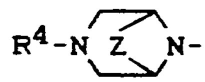, 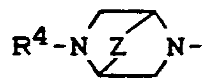 ,

in welcher Z und $R^4$ die in Anspruch 4 angegebene Bedeutung haben, steht,

dadurch gekennzeichnet, daß man eine Verbindung der Formel (IV)

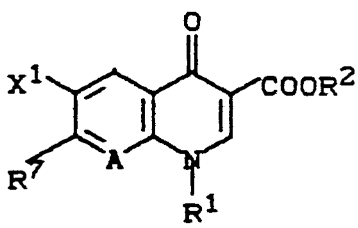

(IV),

in welcher

$R^1$, $R^2$, A und $X^1$ die in Anspruch 5 angegebene Bedeutung haben und

$R^7$ für einen Rest der Formel

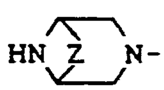 , 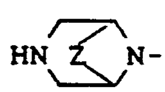 oder 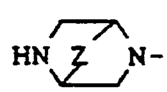 ,

in welcher

Z die oben angegebene Bedeutung hat, steht, mit Verbindungen der Formel (V)

$$R^4-X^3 \quad (V),$$

in welcher

$R^4$ die oben angegebene Bedeutung hat, aber nicht für Wasserstoff stehen kann,

$X^3$ für Halogen, insbesondere Chlor, Brom oder Jod, steht, gegebenenfalls in Gegenwart von Säurebindungsmitteln umsetzt.

8. Verfahren zur Herstellung von 7-(Azabicycloalkyl)-chinoloncarbonsäure- bzw. -naphthyridoncarbonsäure-Derivaten der allgemeinen Formel (I) nach Anspruch 4, worin $R^4$ für $CH_3-CO-CH_2-CH_2-$ steht, durch Umsetzung einer Verbindung der Formel (IV) in Anspruch 7. mit Methylvinylketon.

9. 7-(Azabicycloalkyl)-chinoloncarbonsäure- beziehungsweise -naphthyridoncarbonsäure-Derivate nach Anspruch 2 oder der Formel (I)

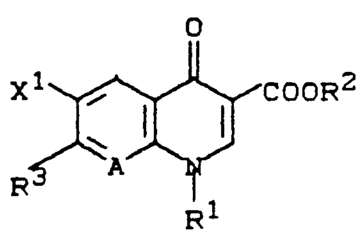

(I),

in welcher

$R^1$ für Methyl, Ethyl, Propyl, Isopropyl, Cyclopropyl, Vinyl, 2-Hydroxyethyl, 2-Fluorethyl, Methoxy, Amino, Methylamino, Dimethylamino, Phenyl, 4-Fluorphenyl, 2,4-Difluorphenyl,

$R^2$ für Wasserstoff, Alkyl mit 1 bis 4 Kohlenstoffatomen, (5-Methyl-2-oxo-1,3-dioxol-4-yl)-methyl,

$R^3$ für einen Rest der Formel

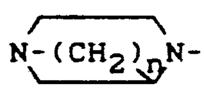 , 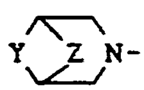 , 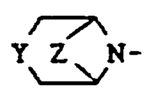 , 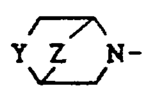 ,

in welcher

Y für $R^4$-N, O, S,

Z für $-(CH_2)_n-$, $-CH_2-O-CH_2-$, $-CH_2-S-CH_2-$, $-CH_2-S-$,

$-CH_2-\overset{\overset{R^5}{|}}{N}-CH_2-$,

n für 1, 2 oder 3,

$R^4$ für Wasserstoff, gegebenenfalls durch Hydroxy substituiertes Alkyl, Alkenyl oder Alkinyl mit

1 bis 4 Kohlenstoffatomen, gegebenenfalls durch Nitro oder Amino substituiertes Benzyl, Oxoalkyl mit 2 bis 4 Kohlenstoffatomen oder (5-Methyl-2-oxo-1,3-dioxol-4-yl)-methyl,

$R^5$ für Wasserstoff oder Methyl steht,

$X^1$ für Fluor, Chlor oder Nitro und

A für N oder C-$R^6$ steht, worin $R^6$ für Wasserstoff, Fluor, Chlor, Methyl oder Nitro steht oder auch gemeinsam mit $R^1$ eine Brücke der Struktur

$-O-CH_2-\underset{|}{CH}-CH_3$, $-S-CH_2-\underset{|}{CH}-CH_3$ oder

$-CH_2-CH_2-\underset{|}{CH}-CH_3$

bilden kann,

mit der Maßgabe, daß für den Fall, daß $X^1$ für Fluor, $R^2$ für H und A für CF oder N stehen und $R^1$ für Ethyl, Cyclopropyl, Fluorethyl oder Vinyl steht oder $R^6$ mit $R^1$ eine Brücke der Struktur $-O-CH_2-\underset{|}{CH}-CH_3$ bildet, n ungleich 2 ist und $R^3$ nicht für den Rest

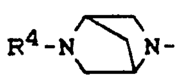 stehen kann, sowie ausgenommen die Verbindung 7-(2,5-Diazabicyclo[2.2.2]oct-2-yl)-1-ethyl-6-fluoro-1,4-dihydro-4-oxo-chinolincarbonsäure,

und deren pharmazeutisch verwendbaren Hydrate und Säureadditionssalze sowie die Alkali-, Erdalkali-, Silber- und Guanidiniumsalze der zugrundeliegenden Carbonsäuren, zur Anwendung in einem Verfahren zur therapeutischen Behandlung des menschlichen oder tierischen Körpers.

10. Arzneimittel enthaltend Verbindungen nach Anspruch 2 oder 7-(Azabicycloalkyl)-chinoloncarbonsäure- bzw. naphthyridoncarbonsäure-Derivate der Formel (I)

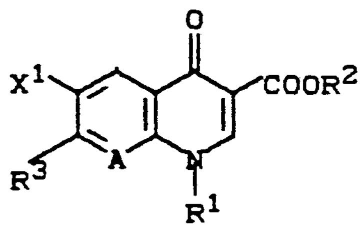

(I),

in welcher

$R^1$ für Methyl, Ethyl, Propyl, Isopropyl, Cyclopropyl, Vinyl, 2-Hydroxyethyl, 2-Fluorethyl, Methoxy, Amino, Methylamino, Dimethylamino, Phenyl, 4-Fluorphenyl, 2,4-Difluorphenyl,

$R^2$ für Wasserstoff, Alkyl mit 1 bis 4 Kohlenstoffatomen, (5-Methyl-2-oxo-1,3-dioxol-4-yl)-methyl,

$R^3$ für einen Rest der Formel

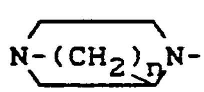 , 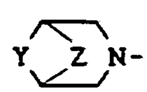 , 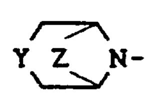 , 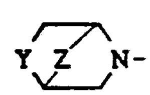 ,

in welcher

Y für $R^4$-N, O, S,

Z für $-(CH_2)_n-$, $-CH_2-O-CH_2-$, $-CH_2-S-CH_2-$, $-CH_2-S-$,

$R^5$
|
$-CH_2-N-CH_2-$,

n für 1, 2 oder 3,

$R^4$ für Wasserstoff, gegebenenfalls durch Hydroxy substituiertes Alkyl, Alkenyl oder Alkinyl mit 1 bis 4 Kohlenstoffatomen, gegebenenfalls durch Nitro oder Amino substituiertes Benzyl, Oxoalkyl mit 2 bis 4 Kohlenstoffatomen oder (5-Methyl-2-oxo-1,3-dioxol-4-yl)-methyl,

$R^5$ für Wasserstoff oder Methyl steht,

$X^1$ für Fluor, Chlor oder Nitro und

A für N oder C-$R^6$ steht, worin
$R^6$ für Wasserstoff, Fluor, Chlor, Methyl oder Nitro steht oder auch gemeinsam mit $R^1$ eine Brücke der Struktur

$-O-CH_2-CH-CH_3$, $-S-CH_2-CH-CH_3$ oder
|

$-CH_2-CH_2-CH-CH_3$
|

bilden kann,

mit der Maßgabe, daß für den Fall, daß $X^1$ für Fluor, $R^2$ für H und A für CF oder N stehen und $R^1$ für Ethyl, Cyclopropyl, Fluorethyl oder Vinyl steht oder $R^6$ mit $R^1$ eine Brücke der Struktur $-O-CH_2-\underset{|}{CH}-CH_3$ bildet, n ungleich 2 ist und $R^3$ nicht für den Rest

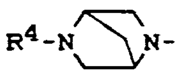 stehen kann, sowie ausgenommen die Verbindung 7-(2,5-Diazabicyclo[2.2.2]oct-2-yl)-1-ethyl-6-fluoro-1,4-dihydro-4-oxo-chinolincarbonsäure,

und deren pharmazeutisch verwendbaren Hydrate und Säureadditionssalze sowie die Alkali-, Erdalkali-, Silber- und Guanidiniumsalze der zugrundeliegenden Carbonsäuren.

11. Verwendung von Verbindungen nach Anspruch 2 oder von 7-(Azabicycloalkyl)-chinoloncarbonsäure- bzw. naphthyridoncarbonsäure-Derivaten der Formel (I)

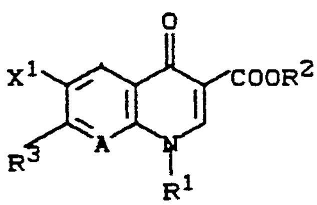

(I),

in welcher

$R^1$ für Methyl, Ethyl, Propyl, Isopropyl, Cyclopropyl, Vinyl, 2-Hydroxyethyl, 2-Fluorethyl, Methoxy, Amino, Methylamino, Dimethylamino, Phenyl, 4-Fluorphenyl, 2,4-Difluorphenyl,

$R^2$ für Wasserstoff, Alkyl mit 1 bis 4 Kohlenstoffatomen, (5-Methyl-2-oxo-1,3-dioxol-4-yl)-methyl,

$R^3$ für einen Rest der Formel

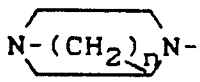 , 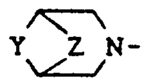 , 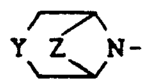 , 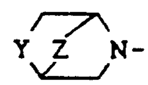 ,

in welcher

Y für $R^4$-N, O, S,

Z für $-(CH_2)_n-$, $-CH_2-O-CH_2-$, $-CH_2-S-CH_2-$, $-CH_2-S-$,

$R^5$
|
$-CH_2-N-CH_2-$,

n für 1, 2 oder 3,

$R^4$ für Wasserstoff, gegebenenfalls durch Hydroxy substituiertes Alkyl, Alkenyl oder Alkinyl mit 1 bis 4 Kohlenstoffatomen, gegebenenfalls durch Nitro oder Amino substituiertes Benzyl, Oxoalkyl mit 2 bis 4 Kohlenstoffatomen oder (5-Methyl-2-oxo-1,3-dioxol-4-yl)-methyl,

$R^5$ für Wasserstoff oder Methyl steht,

$X^1$ für Fluor, Chlor oder Nitro und

A für N oder C-$R^6$ steht, worin $R^6$ für Wasserstoff, Fluor, Chlor, Methyl oder Nitro steht oder auch gemeinsam mit $R^1$ eine Brücke der Struktur

$-O-CH_2-\underset{|}{CH}-CH_3$, $-S-CH_2-\underset{|}{CH}-CH_3$ oder

$-CH_2-CH_2-\underset{|}{CH}-CH_3$

bilden kann,

mit der Maßgabe, daß für den Fall, daß $X^1$ für Fluor, $R^2$ für H und A für CF oder N stehen und $R^1$ für Ethyl, Cyclopropyl, Fluorethyl oder Vinyl steht oder $R^6$ mit $R^1$ eine Brücke der Struktur $-O-CH_2-\underset{|}{CH}-CH_3$ bildet, n ungleich 2 ist und $R^3$ nicht für den Rest

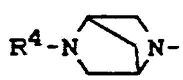 stehen kann, sowie ausgenommen die Verbindung 7-(2,5-Diazabicyclo[2.2.2]oct-2-yl)-1-ethyl-6-fluoro-1,4-dihydro-4-oxo-chinolincarbonsäure,

und deren pharmazeutisch verwendbaren Hydrate und Säureadditionssalze sowie die Alkali-, Erdalkali-, Silber- und Guanidiniumsalze der zugrundeliegenden Carbonsäuren,

zur Herstellung von Arzneimitteln.

12. Tierfutter oder Tierfutterzusätze, enthaltend 7-(Azabicycloalkyl)-chinoloncarbonsäure- beziehungsweise -naphthyridoncarbonsäure-Derivate der Formel (I)

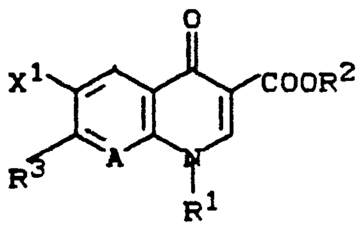

(I),

in welcher

$R^1$ für Methyl, Ethyl, Propyl, Isopropyl, Cyclopropyl, Vinyl, 2-Hydroxyethyl, 2-Fluorethyl, Methoxy, Amino, Methylamino, Dimethylamino, Phenyl, Fluorphenyl, 2,4-Difluorphenyl,

$R^2$ für Wasserstoff, Alkyl mit 1 bis 4 Kohlenstoffatomen, (5-Methyl-2-oxo-1,3-dioxol-4-yl)-methyl,

$R^3$ für einen im Ringsystem gegebenenfalls durch Hydroxy oder Methyl ein- oder mehrfach substituierten Rest der Formel

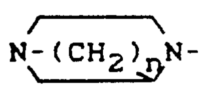 , 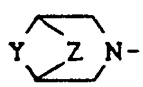 , 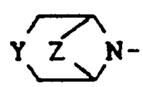 , 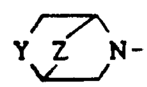 ,

in welcher

Y für $R^4$-N, O, S,

Z für $-(CH_2)_n-$, $-CH_2-O-CH_2-$, $-CH_2-S-CH_2-$, $-CH_2-S-$,

$R^5$
|
$-CH_2-N-CH_2-$,

n für 1, 2 oder 3,

$R^4$ für Wasserstoff, gegebenenfalls durch Hydroxy substituiertes Alkyl, Alkenyl oder Alkinyl mit 1 bis 4 Kohlenstoffatomen, gegebenenfalls durch Nitro oder Amino substituiertes Benzyl, Oxoalkyl mit 2 bis 4 Kohlenstoffatomen oder (5-Methyl-2-oxo-1,3-dioxol-4-yl)-methyl und

$R^5$ für Wasserstoff oder Methyl steht,

$X^1$ für Fluor, Chlor oder Nitro und

A für N oder C-$R^6$ steht, worin
$R^6$ für Wasserstoff, Fluor, Chlor, Methyl oder Nitro steht oder auch gemeinsam mit $R^1$ eine Brücke der Struktur

$-O-CH_2-CH-CH_3$, $-S-CH_2-CH-CH_3$ oder
|  |

$-CH_2-CH_2-CH-CH_3$
|

bilden kann,

und deren pharmazeutisch verwendbaren Hydrate und Säureadditionssalze sowie die Alkali-, Erdalkali-, Silber- und Guanidiniumsalze der zugrundeliegenden Carbonsäuren.

13. 7-Chlor-1-cyclopropyl-6-fluor-1,4-dihydro-8-nitro-4-oxo-3-chinolincarbonsäure.

14. 5-Methyl-1,4-diazabicyclo[3.2.1]octan.